# EUROPÄISCHE PATENTSCHRIFT

(11) **EP 2 928 887 B1**
(45) Veröffentlichungstag und Bekanntmachung des Hinweises auf die Patenterteilung: **16.11.2016**
(21) Anmeldenummer: 13799056.0
(22) Anmeldetag: 03.12.2013
(51) Int. Cl.: C07D 413/12, C07D 417/14, C07D 263/48, A01N 43/76, A01N 43/82

(54) **N-(OXAZOL-2-YL)-ARYLCARBONSÄUREAMIDE UND IHRE VERWENDUNG ALS HERBIZIDE**
N-(OXAZOL-2-YL)ARYL CARBOXYLIC ACID AMIDES AND USE OF SAME AS HERBICIDES
AMIDES D'ACIDE N-(OXAZOL-2-YL)-ARYLE CARBONIQUE ET LEUR UTILISATION COMME HERBICIDES

(30) Priorität: 06.12.2012 EP 12195959
(43) Veröffentlichungstag der Anmeldung: 14.10.2015
(73) Patentinhaber: Bayer CropScience AG, 40789 Monheim am Rhein (DE)
(72) Erfinder: KÖHN, Arnim, 55270 Klein-Winternheim (DE); AHRENS, Hartmut, 63329 Egelsbach (DE); BRAUN, Ralf, 76857 Ramberg (DE); HEINEMANN, Ines, 65719 Hofheim (DE); TIEBES, Jörg, 60431 Frankfurt (DE); WALDRAFF, Christian, 61118 Bad Vilbel (DE); DIETRICH, Hansjörg, 65835 Liederbach am Taunus (DE); GATZWEILER, Elmar, 61231 Bad Nauheim (DE); ROSINGER, Christopher Hugh, 65719 Hofheim (DE); SCHMUTZLER, Dirk, 65795 Hattersheim (DE)
(74) Vertreter: BIP Patents
(86) Internationale Anmeldenummer: PCT/EP2013/075297
(87) Internationale Veröffentlichungsnummer: WO 2014/086734

(56) Entgegenhaltungen:
- WO-A1-2010/132404
- WO-A1-2011/035874
- WO-A1-2012/028579
- JP-A- 64 009 978
- US-A- 6 096 688

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

WO 2011/035874 A1 offenbart N-(1,2,5-Oxadiazol-3-yl)benzamide und ihre Verwendung als Herbizide. WO 2012/028579 A1 offenbart N-(Tetrazol-5-yl)- und N-(Triazol-5-yl)arylcarbonsäureamide und ihre Verwendung als Herbizide

Die aus diesen Schriften bekannten Verbindungen zeigen jedoch keine oder häufig eine nicht ausreichende herbizide Wirksamkeit. Aufgabe der vorliegenden Erfindung ist daher die Bereitstellung von weiteren herbizid wirksamen Verbindungen.

In WO 2010/132404 A1 wird die pharamakologisch wirksame Verbindung {[(5-Methoxy-2-{[5-(2,2,2-trifluorethyl)-1,3-oxazol-2-yl]carbamoyl}phenoxy)carbonyl] oxy}methyl-2,2-dimethylpropanoat beschrieben.
Unter den nachfolgenden CAS-Nummern sind jeweils die danach genannten Verbindungen bekannt.
1187436-88-9: Ethyl-4-methyl-2-({[2-methyl-6-(trifluormethyl)pyridin-3-yl]carbonyl}amino)-1,3-oxazol-5-carboxylat.
1090036-46-6: N-(4,5-Dimethyl-1,3-oxazol-2-yl)-2,4-dimethylbenzamid. 587008-52-4: 2,4-Dichlor-N-(4,5-diphenyl-1,3-oxazol-2-yl)benzamid.
Eine herbizide Wirkung der über ihre CAS-Nummern bekannten Verbindungen ist nicht offenbart.

Es wurde nun gefunden, dass N-(1,3-Oxazol-2-yl)-arylcarbonsäureamide, die im Arylcarbonsäureteil bestimmte Substituenten tragen, als Herbizide besonders gut geeignet sind.

Ein Gegenstand der vorliegenden Erfindung sind somit N-(1,3-Oxazol-2-yl)-arylcarbonsäureamide der Formel (I) oder deren Salze 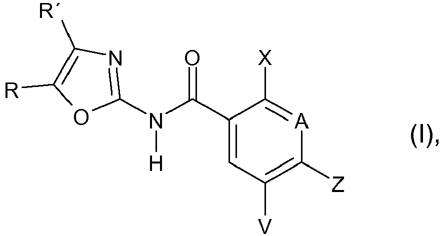 worin
A bedeutet N oder CY,
R und R' bedeuten unabhängig voneinander jeweils Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, (C₂-C₆)-Halogenalkenyl, (C₂-C₆)-Alkinyl, (C₂-C₆)-Alkinyloxy, (C₂-C₆)-Halogenalkinyl, Cyano-(C₁-C₆)-Alkyl, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe Methyl, Ethyl, Methoxy, Trifluormethyl und Halogen substituiertes Heteroaryl, Heterocyclyl oder Phenyl,
X bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-CyCloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OR², OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, CH₂P(O)(OR⁵)₂, (C₁-C₆)-Alkyl-Heteroaryl, (C₁-C₆)-Alkyl-Heterocyclyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-Cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, CHNOR¹, CH₂ONCR³)₂, NR¹SO₂R², NR¹COR¹, OR¹, OSO₂R², S(O)ₙR², SO₂OR¹, SO₂N(R¹)₂(C₁-C₆)-Alkyl-S(O)ₙR², NS(O)R⁶R⁷, S(O)R⁸NR⁹, (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R ², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, Nitro, (C₁-C₆)-Alkyl, (C₁-C₆)-Alkoxy, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR¹R², P(O)(OR⁵)₂, Heteroaryl, Heterocyclyl oder Phenyl, wobei die letzten drei Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl 0 bis 2 Oxogruppen trägt, oder
Z kann auch Wasserstoff bedeuten, falls Y für den Rest S(O)ₙR² steht,
V bedeutet Wasserstoff, (C₁-C₆)-Alkyl, Halogen-(C₁-C₆)-alkyl, (C₁-C₆)-Alkoxy, (C₁-C₆)-Halogenalkoxy, S(O)ₙ-(C₁-C₆)-Alkyl, S(O)ₙ-(C₁-C₆)-Halogenalkyl, (C₁-C₆)-Alkoxy-(C₁-C₄)-alkyl, Halogen, Nitro oder Cyano,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(OₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Methyl oder Ethyl,
R⁶ und R⁷ bedeuten unabhängig voneinander jeweils (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy-(C₁-C₆)-alkyl, Halogen-(C₁-C₆)-alkoxy-(C₁-C₆)-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
   oder
R⁶ und R⁷ bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außerdem Schwefelatom der Sulfoximinogruppe jeweils m Ringglieder aus der Gruppe bestehend aus N(R¹), O und S(O)ₙ enthält, und wobei dieser Ring jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl, substituiert ist, und wobei dieser Ring n Oxogruppen trägt,
R⁸ bedeutet jeweils durch s Reste aus der Gruppe bestehend aus Nitro, Halogen, Cyano, Rhodano, (C₃-C₆)-Cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S und (R⁵O)₂(O)P substituiertes (C₁-C₆)-Alkyl, (C₂-C₆)-Alkenyl oder (C₂-C₆)-Alkinyl,
   oder
jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO,
(R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P und R¹O-(C₁-C₆)-Alkyl im cyclischen Teil substituiertes (C₃-C₆)-Cycloalkyl, (C₃-C₆)-Cycloalkenyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R¹)-(C₁-C₆)-alkyl, Heteroaryl- N(R¹)-(C₁-C₆)-alkyl, Heterocyclyl- N(R¹)-(C₁-C₆)-alkyl, Phenyl-S(O)ₙ-(C₁-C₆)-alkyl, Heteroaryl-S(O)ₙ-(C₁-C₆)-alkyl oder Heterocyclyl-S(O)ₙ-(C₁-C₆)-alkyl, und wobei Heterocyclyl n Oxogruppen trägt,
R⁹ bedeutet Wasserstoff, Nitro, Halogen, Cyano, (C₁-C₆)-Alkyl, Halogen-(C₁-C₆)-alkyl, (C₃-C₆)-Alkenyl, Halogen-(C₃-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-Cycloalkyl-(C₁-C₆)-alkyl, R¹(O)C, R²O(O)C, (R¹)₂N(O)C, R²S(O)C, (R¹)₂N(S)C, R¹(R¹O)N(O)C, R²(O)₂S(R¹)N(O)C, (R¹)₂N(O)₂S(R¹)N(O)C, R¹O, (R¹)₂N, R²(O)ₙS, (R²)₃Si-(C₁-C₆)-Alkyl-(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, R²(O)₂S(R¹)N(O)₂S, (R⁵O)₂(O)P, (R²)₃Si, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, (R²)₃Si-(C₁-C₆)-Alkyl,
   oder
jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl im cyclischen Teil substituiertes Phenyl, Heteroaryl, Heterocyclyl, Phenyl-(C₁-C₆)-alkyl, Heteroaryl-(C₁-C₆)-alkyl oder Heterocyclyl-(C₁-C₆)-alkyl, und wobei Heterocyclyl n Oxogruppen trägt,
m bedeutet 0, 1, 2, 3 oder 4,
n bedeutet 0, 1 oder 2,
s bedeutet 0, 1, 2 oder 3,
wobei die Verbindungen {[(5-Methoxy-2-{[5-(2,2,2-trifluorethyl)-1,3-oxazol-2-yl]carbamoyl}phenoxy)carbonyl]oxy}methyl-2,2-dimethylpropanoat, Ethyl-4-methyl-2-({[2-methyl-6-(trifluormethyl)pyridin-3-yl]carbonyl}amino)-1,3-oxazol-5-carboxylat, N-(4,5-Dimethyl-1,3-oxazol-2-yl)-2,4-dimethylbenzamid und 2,4-Dichlor-N-(4,5-diphenyl-1,3-oxazol-2-yl)benzamid ausgenommen sind.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1 H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Bevorzugt sind Verbindungen der allgemeinen Formel (I), worin
A bedeutet N oder CY,
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, C₁-C₆)-Alkyl-O-(C₁-C₆)-alkyl, (C₃-C₆)-Cycloalkyl-(C₁-C₆)-alkyl, Halogen-(C₃-C₆)-cycloalkyl-(C₁-C₆)-alkyl, COR¹, OR², OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹ oder (C₁-C₆)-Alkyl-NR¹SO₂R², (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-Cycloalkyl-(C₁-C₆)-alkyl, COR¹, OR¹, COOR¹, OSO₂R², S(O)ₙR², SO₂OR¹, SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, Nitro, (C₁-C₆)-Alkyl, (C₁-C₆)-Alkoxy, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-Cycloalkyl-(C₁-C₆)-alkyl, COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², 1,2,4-Triazol-1-yl, oder
Z kann auch Wasserstoff bedeuten, falls Y für den Rest S(O)ₙR² steht,
V bedeutet Wasserstoff, (C₁-C₆)-Alkyl, Halogen-(C₁-C₆)-alkyl, (C₁-C₆)-Alkoxy, (C₁-C₆)-Halogenalkoxy, S(O)ₙ-(C₁-C₆)-alkyl, S(O)ₙ-(C₁-C₆)-halogenalkyl, (C₁-C₆)-Alkoxy-(C₁-C₄)-alkyl, Halogen, Nitro oder Cyano,
R, R' bedeuten unabhängig voneinander jeweils Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano-(C₁-C₆)-Alkyl, Cyano, Methylsulfenyl, Methylsulfinyl, Methylsulfonyl, Acetylamino, Methoxymethyl, oder
jeweils durch s Reste aus der Gruppe Methyl, Ethyl, Methoxy, Trifluormethyl und Halogen substituiertes Heteroaryl, Heterocyclyl oder Phenyl,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei diese Reste durch s Reste aus der Gruppe bestehend aus Cyano, Halogen, Nitro, OR³, S(O)_{N}R⁴, N(R³)₂, NR³OR³, NR³SO₂R⁴, COR³, OCOR³, NR³COR³, CO₂R³, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl oder (C₂-C₆)-Alkinyl,
R⁶ und R⁷ bedeuten unabhängig voneinander jeweils (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy-(C₁-C₆)-alkyl, Halogen-(C₁-C₆)-alkoxy-(C₁-C₆)-alkyl, Phenyl, Heteroaryl oder Heterocyclyl, wobei die drei letztgenannten Reste jeweils durch s Reste aus der Gruppe bestehend aus Nitro, Halogen, (C₁-C₆)-Alkyl, Halogen-(C₁-C₆)-alkyl, R¹O(O)C, (R¹)₂N(O)C, R¹O, (R¹)₂N, R²(O)ₙS, und R¹O-(C₁-C₆)-Alkyl substituiert sind, und wobei Heterocyclyl n Oxogruppen trägt,
oder R⁶ und R⁷ bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außerdem Schwefelatom der Sulfoximinogruppe jeweils m Ringglieder aus der Gruppe bestehend aus N(R¹), O und S(O)ₙ enthält, und wobei dieser Ring jeweils durch s Reste aus der Gruppe bestehend aus Halogen, (C₁-C₆)-Alkyl, Halogen-(C₁-C₆)-alkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl, substituiert ist, und wobei dieser Ring n Oxogruppen trägt,
R⁸ bedeutet jeweils durch s Reste aus der Gruppe bestehend aus Halogen, Cyano, (C₃-C₆)-Cycloalkyl, R¹(O)C, R¹(R¹ON=)C, R¹O(O)C, (R¹)₂N(O)C, R²(O)₂S(R¹)N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, R¹(O)C(^{R}1)N(O)₂S, R²O(O)C(R¹)N(O)₂S und (R¹)₂N(O)C(R¹)N(O)₂S substituiertes (C₁-C₆)-Alkyl oder
jeweils durch s Reste aus der Gruppe bestehend aus Halogen, (C₁-C₆)-Alkyl, Halogen-(C₁-C₆)-alkyl, (C₃-C₆)-Cycloalkyl, R¹O(O)C und (R¹)₂N(O)C substituiertes (C₃-C₆)-Cycloalkyl,
R⁹ bedeutet Wasserstoff, Nitro, Cyano, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R²O(O)C, (R¹)₂N(O)C, R²(O)₂S, R¹(O)C-(C₁-C₆)-Alkyl, R¹O(O)C-(C₁-C₆)-Alkyl, (R¹)₂N(O)C-(C₁-C₆)-Alkyl, R¹O-(C₁-C₆)-Alkyl, (R¹)₂N-(C₁-C₆)-Alkyl oder R²(O)ₙS-(C₁-C₆)-Alkyl,
- m: bedeutet 0, 1 oder 2,
- n: bedeutet 0, 1 oder 2,
- s: bedeutet 0, 1, 2 oder 3.
Besonders bevorzugt sind Verbindungen der allgemeinen Formel (I), worin A bedeutet N oder CY,
X bedeutet Nitro, Halogen, Cyano, (C₁-C₆)-Alkyl, Halogen-(C₁-C₆)-alkyl, (C₃-C₆)-Cycloalkyl, OR², S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)ₙR², SO_{2N}(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Nitro, (C₁-C₆)-Alkyl, (C₁-C₆)-Alkoxy, Halogen-(C₁-C₆)-alkyl, (C₃-C₆)-Cycloalkyl, S(O)ₙR², 1,2,4-Triazol-1-yl, oder Z kann auch Wasserstoff, bedeuten, falls Y für den Rest S(O)ₙR² steht,
V bedeutet Wasserstoff, (C₁-C₆)-Alkyl, Halogen-(C₁-C₆)-alkyl, (C₁-C₆)-Alkoxy, (C₁-C₆)-Halogenalkoxy, S(O)ₙ-(C₁-C₆)-alkyl, S(O)ₙ-(C₁-C₆)-halogenalkyl, (C₁-C₆)-Alkoxy-(C₁-C₄)-alkyl, Halogen, Nitro oder Cyano,
R, R' bedeuten unabhängig von einander jeweils Wasserstoff, (C₁-C₆)-Alkyl, (C₃-C₇)-cyclo-Alkyl, Halogen-(C₁-C₆)-alkyl,(C₁-C₆)-Alkoxy, Halogen-(C₁-C₆)-alkoxy, Cyano-(C₁-C₆)-Alkyl, Cyano, Methylsulfenyl, Methylsulfinyl, Methylsulfonyl, Acetylamino, Halogen, Amino, Methoxymethyl,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₃-C₆)-Cycloalkyl oder (C₃-C₆)-Cycloalkyl-(C₁-C₆)-alkyl, wobei diese drei vorstehend genannten Reste jeweils durch s Reste aus der Gruppe bestehend aus Halogen und OR³ substituiert sind,
R³ bedeutet Wasserstoff oder (C₁-C₆)-Alkyl,
R⁴ bedeutet (C₁-C₆)-Alkyl,
R⁶ und R⁷ bedeuten unabhängig voneinander jeweils Methyl, Ethyl oder n-Propyl, oder
R⁶ und R⁷ bilden gemeinsam mit dem Schwefel-Atom, an dem sie gebunden sind, einen 5- oder 6-gliedrigen gesättigten Ring, der außer den Kohlenstoffatomen und außer dem Schwefelatom der Sulfoximinogruppe m Sauerstoffatome enthält,
- R⁸: bedeutet Methyl, Ethyl oder n-Propyl,
- R⁹: bedeutet Wasserstoff oder Cyano,
- m: bedeutet 0 oder 1,
- n: bedeutet 0, 1 oder 2,
- s: bedeutet 0, 1, 2 oder 3.

In allen nachfolgend genannten Formeln haben die Substituenten und Symbole, sofern nicht anders definiert, dieselbe Bedeutung wie unter Formel (I) beschrieben. Erfindungsgemäße Verbindungen können beispielsweise nach der in Schema 1 angegebenen Methode durch basenkatalysierte Umsetzung eines Benzoesäurechlorids (II) mit einem 2-Amino-1,3-oxazol (III) hergestellt werden:

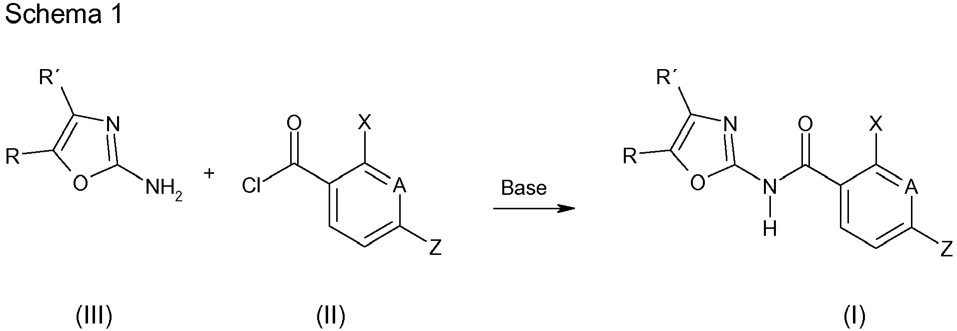

Erfindungsgemäße Verbindungen können auch nach der in Schema 2 angegebenen Methode durch Umsetzung einer Benzoesäure der Formel (IV) mit einem 2-Amino-1,3-oxazol (III) hergestellt werden:

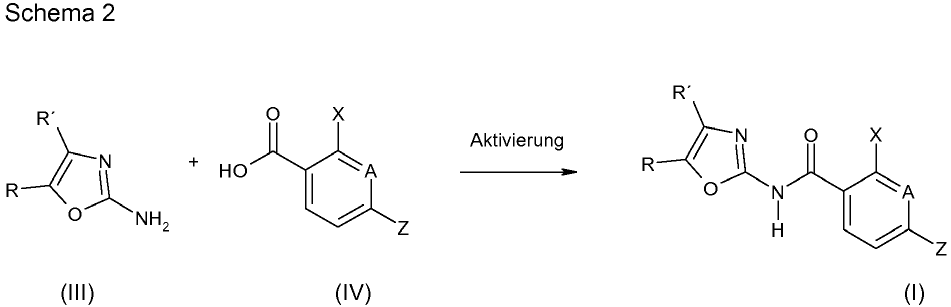

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.
Es kann zweckmäßig sein, Reaktionsschritte in ihrer Reihenfolgezu ändern.So sind Benzoesäuren, die ein Sulfoxid tragen, nicht ohne weiteres in ihre Säurechloride zu überführen. Hier bietet sich an, zunächst auf Thioether-Stufe das Amid zu herzustellen und danach den Thioether zum Sulfoxid zu oxidieren.
Die Benzoesäurechloride der Formel (II) beziehungsweise die ihnen zugrunde liegenden Benzoesäuren (IV) sind grundsätzlich bekannt und können beispielsweise gemäß den in US 6,376,429 B1, EP 1 585 742 A1 und EP 1 202 978 A1 beschriebenen Methoden hergestellt werden.

Die 2-Amino-1,3-oxazole der Formel (III) sind entweder käuflich erhältlich oder können analog zu literaturbekannten Methoden hergestellt werden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of

Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Synthese von 2-Chlor-4-(methylsulfonyl)-N-(1,3-oxazol-2-yl)-3-[(2,2,2-trifluorethoxy)methyl]benzamid, (Tabellenbeispiel Nr. 1-334)

347 mg (1,0 mmol) 2-Chlor-4-(methylsulfonyl)-3-[(2,2,2-trifluorethoxy) methyl]benzoesäure und 84 mg (1,0 mmol) 1,3-Oxazol-2-amin werden bei Raumtemperatur (RT) in 7 ml Dichlormethan gelöst und mit 0,14 ml (1,0 mmol) Triethylamin, 24 mg (0,20 mmol) DMAP und 955 mg (1,5 mmol) 2,4,6-Tripropyl-1,3,5,2,4,6-trioxatriphosphinan-2,4,6-trioxid (50%ige Lösung in THF) versetzt. Das Reaktionsgemisch wird 20 h bei RT gerührt und anschließend zweimal mit jeweils 5 ml Wasser gewaschen. Die organische Phase wird über Na₂SO₄ getrocknet und eingedampft. Der Rückstand wird säulenchromatographisch (HPLC, Acetonitril / Wasser) gereinigt. Ausbeute 93 mg (20%).

### Synthese von 2-Chlor-N-(4-methyl-1,3-oxazol-2-yl)-4-(methylsulfonyl)benzamid, (Tabellenbeispiel Nr. 7-13)

574 mg (2,0 mmol) 2-Chlor-4-methylsulfonyl-benzoesäure und 240 mg (1,0 mmol) 4-Methyl-1,3-oxazol-2-amin werden bei RT in 7 ml Dichlormethan gelöst und mit 0,34 ml (2,0 mmol) Triethylamin, 60 mg (0,49 mmol) DMAP und 2,335 g (4 mmol) 2,4,6-Tripropyl-1,3,5,2,4,6-trioxatriphosphinan-2,4,6-trioxid (50%ige Lösung in THF) versetzt. Das Reaktionsgemisch wird 20 h bei RT gerührt und anschließend zweimal mit jeweils 5 ml Wasser gewaschen. Die organische Phase wird über Na₂SO₄ getrocknet und eingedampft. Der Rückstand wird säulenchromatographisch (HPLC, Acetonitril / Wasser) gereinigt. Ausbeute 66 mg (7,3 %).

### Synthese von 2-Chlor-N-(4-phenyl-1,3-oxazol-2-yl)-4-(methylsulfonyl)benzamid, (Tabellenbeispiel Nr. 9-13)

293 mg (1,25 mmol) 2-Chlor-4-methylsulfonyl-benzoesäure und 200 mg (1,25 mmol) 4-Phenyl-1,3-oxazol-2-amin werden bei RT in 7 ml Dichlormethan gelöst und mit 0,17 ml (1,25 mmol) Triethylamin, 31 mg (0,25 mmol) DMAP und 1,19 g (1,88 mmol) 2,4,6-Tripropyl-1,3,5,2,4,6-trioxatriphosphinan-2,4,6-trioxid (50%ige Lösung in THF) versetzt. Das Reaktionsgemisch wird 20 h bei RT gerührt und anschließend zweimal mit jeweils 5 ml Wasser gewaschen. Die organische Phase wird über Na₂SO₄ getrocknet und eingedampft. Der Rückstand wird säulenchromatographisch (HPLC, Acetonitril / Wasser) gereinigt. Ausbeute 91 mg (17 %).

### Synthese von 2-Chlor-N-(1,3-oxazol-2-yl)-6-(trifluormethyl)nicotinamid, (Tabellenbeispiel Nr. 10-1)

537 mg (2,38 mmol) 2-Chlor-6-(trifluormethyl)nicotinsäureund 200 mg (2,38 mmol) 1,3-Oxazol-2-amin werden bei RT in 7 ml Dichlormethan gelöst und mit 0,33 ml (2,38 mmol) Triethylamin, 58 mg (0,47 mmol) DMAP und 2,27 g (3,57 mmol) 2,4,6-Tripropyl-1,3,5,2,4,6-trioxatriphosphinan-2,4,6-trioxid (50%ige Lösung in THF) versetzt. Das Reaktionsgemisch wird 20 h bei RT gerührt und anschließend zweimal mit jeweils 5 ml Wasser gewaschen. Die organische Phase wird über Na₂SO₄ getrocknet und eingedampft. Der Rückstand wird säulenchromatographisch (HPLC, Acetonitril / Wasser) gereinigt. Ausbeute 40 mg (5%).

Die in den nachfolgenden Tabellen aufgeführten Beispiele wurden analog oben genannten Methoden hergestellt beziehungsweise sind analog oben genannten Methoden erhältlich. Die in den nachfolgenden Tabellen aufgeführten Verbindungen sind ganz besonders bevorzugt.

Die verwendeten Abkürzungen und Bezeichnungen bedeuten:
Et = Ethyl Me = Methyl n-Pr = n-Propyl c-Pr = cyclo-Propyl i-Pr = iso-Propyl Bn = Benzyl Ph = Phenyl Ac = Acetyl t-Bu = tertiär-Butyl

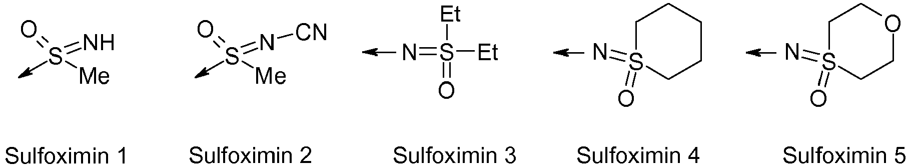

**Tabelle 1: Erfindungsgemäße Verbindungen der allgemeinen Formel (I), worin R und R' für H, und A für C-Y steht.**

| | | | | | |
|---|---|---|---|---|---|
| 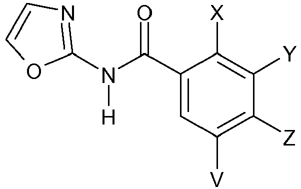 | | | | | |

| Nr. | X | Y | Z | V | Physikalische Daten (¹H-NMR, DMSO-d₆, 400 MHz) |
|---|---|---|---|---|---|
| 1-1 | F | H | F | H | |
| 1-2 | F | H | Cl | H | |
| 1-3 | F | H | SO₂Me | H | |
| 1-4 | F | H | SO₂Et | H | |
| 1-5 | F | H | CF₃ | H | |
| 1-6 | F | H | NO₂ | H | |
| 1-7 | Cl | H | F | H | |
| 1-8 | Cl | H | F | F | |
| 1-9 | Cl | H | Cl | H | |
| 1-10 | Cl | H | Br | H | |
| 1-11 | Cl | H | SMe | H | |
| 1-12 | Cl | H | SOMe | H | |
| 1-13 | Cl | H | SO₂Me | H | |
| 1-14 | Cl | H | SO₂Et | H | |
| 1-15 | Cl | H | CF₃ | H | |
| 1-16 | Cl | H | NO₂ | H | |
| 1-17 | Cl | H | pyrazol-1-yl | H | |
| 1-18 | Br | H | Cl | H | |
| 1-19 | Br | H | Br | H | |
| 1-20 | Br | H | SO₂Me | H | |
| 1-21 | Br | H | SO₂Et | H | |
| 1-22 | Br | H | CF₃ | H | |
| 1-23 | SO₂Me | H | Cl | H | |
| 1-24 | SO₂Me | H | Br | H | |
| 1-25 | SO₂Me | H | SMe | H | |
| 1-26 | SO₂Me | H | SOMe | H | |
| 1-27 | SO₂Me | H | SO₂Me | H | |
| 1-28 | SO₂Me | H | SO₂Et | H | |
| 1-29 | SO₂Me | H | CF₃ | H | |
| 1-30 | SO₂Et | H | Cl | H | |
| 1-31 | SO₂Et | H | Br | H | |
| 1-32 | NO₂ | H | NO₂ | H | |
| 1-33 | SO₂Et | H | SO₂Me | H | |
| 1-34 | SO₂Et | H | CF₃ | H | |
| 1-35 | CH₂SO₂Me | H | Br | H | |
| 1-36 | CH₂SO₂Me | H | CF₃ | H | 12.03 (s, 1 H), 7.95 - 7.86 (m, 4H), 7.17 (s, 1H), 4.97 (s, 2H), 2.96 (s, 3H) |
| 1-37 | NO₂ | H | F | H | |
| 1-38 | NO₂ | H | Cl | H | |
| 1-39 | NO₂ | H | Br | H | |
| 1-40 | NO₂ | H | I | H | |
| 1-41 | NO₂ | H | CN | H | |
| 1-42 | NO₂ | H | SO₂Me | H | |
| 1-43 | NO₂ | H | SO₂Et | H | |
| 1-44 | NO₂ | H | CF₃ | H | |
| 1-45 | Me | H | F | H | |
| 1-46 | Me | H | Cl | H | |
| 1-47 | Me | H | Br | H | |
| 1-48 | Me | H | I | H | |
| 1-49 | Me | H | CN | H | |
| 1-50 | Me | H | SO₂Me | H | |
| 1-51 | Me | H | SO₂Et | H | |
| 1-52 | Me | H | CF₃ | H | |
| 1-53 | Et | H | F | H | |
| 1-54 | Et | H | Cl | H | |
| 1-55 | Et | H | Br | H | |
| 1-56 | Et | H | I | H | |
| 1-57 | Et | H | CN | H | |
| 1-58 | Et | H | SO₂Me | H | |
| 1-59 | Et | H | SO₂Et | H | |
| 1-60 | Et | H | CF₃ | H | |
| 1-61 | CF₃ | H | NO₂ | H | |
| 1-62 | CF₃ | H | Br | H | |
| 1-63 | CF₃ | H | CF₃ | H | |
| 1-64 | CF₃ | H | SO₂Me | H | |
| 1-65 | CF₃ | H | SO₂Et | H | |
| 1-66 | CF₃ | H | Cl | H | |
| 1-67 | NO₂ | NH₂ | F | H | |
| 1-68 | NO₂ | NHMe | F | H | |
| 1-69 | NO₂ | NMe₂ | F | H | |
| 1-70 | NO₂ | Me | Cl | H | |
| 1-71 | NO₂ | NH₂ | Cl | H | |
| 1-72 | NO₂ | NHMe | Cl | H | |
| 1-73 | NO₂ | NMe₂ | Cl | H | |
| 1-74 | NO₂ | NH₂ | Br | H | |
| 1-75 | NO₂ | NHMe | Br | H | |
| 1-76 | NO₂ | NMe₂ | Br | H | |
| 1-77 | NO₂ | NH₂ | CF₃ | H | |
| 1-78 | NO₂ | NMe₂ | CF₃ | H | |
| 1-79 | NO₂ | NH₂ | SO₂Me | H | |
| 1-80 | NO₂ | NH₂ | SO₂Et | H | |
| 1-81 | NO₂ | NHMe | SO₂Me | H | |
| 1-82 | NO₂ | NMe₂ | SO₂Me | H | |
| 1-83 | NO₂ | NMe₂ | SO₂Et | H | |
| 1-84 | NO₂ | NH₂ | 1H-1,2,4-triazol-1-yl | H | |
| 1-85 | NO₂ | NHMe | 1H-1,2,4-triazol-1-yl | H | |
| 1-86 | NO₂ | NMe₂ | 1H-1,2,4-triazol-1-yl | H | |
| 1-87 | Me | F | F | H | |
| 1-88 | Me | F | Cl | H | |
| 1-89 | Me | Me | SO₂Me | H | |
| 1-90 | Me | F | SO₂Me | H | |
| 1-91 | Me | Cl | Cl | H | |
| 1-92 | Me | O(CH₂)₂OMe | Cl | H | |
| 1-93 | Me | O(CH₂)₃OMe | Cl | H | |
| 1-94 | Me | O(CH₂)₄OMe | Cl | H | |
| 1-95 | Me | OCH₂CONMe₂ | Cl | H | |
| 1-96 | Me | O(CH₂)₂-CO-NMe₂ | Cl | H | |
| 1-97 | Me | O(CH₂)₂-NH(CO)NMe₂ | Cl | H | |
| 1-98 | Me | O(CH₂)₂-NH(CO)NHCO₂Et | Cl | H | |
| 1-99 | Me | O(CH₂)₂-NHCO₂Me | Cl | H | |
| 1-100 | Me | OCH₂-NHSO₂cPr | Cl | H | |
| 1-101 | Me | O(CH₂)-5-2,4-dimethyl-2,4-dihydro-3H-1,2,4-triazol-3-on | Cl | H | |
| 1-102 | Me | O(CH₂)-3,5-dime-thyl-1,2-oxazol-4-yl | Cl | H | |
| 1-103 | Me | OCH₂(CO)NMe₂ | Br | H | |
| 1-104 | Me | O(CH₂)-5-pyrrolidin-2-on | Br | H | |
| 1-105 | Me | Cl | CF₃ | H | |
| 1-106 | Me | Me | SO₂Me | H | |
| 1-107 | Me | 4,5-dihydro-1,2-oxazol-3 yl | SO₂Me | H | |
| 1-108 | Me | 4,5-dihydro-1,2-oxazol-3 yl | SO₂Et | H | |
| 1-109 | Me | 5-cyanomethyl- 4,5-dihydro-1,2-oxazol-3-yl | SO₂Me | H | |
| 1-110 | Me | 5-cyanomethyl- 4,5-dihydro-1,2-oxazol-3-yl | SO₂Et | H | |
| 1-111 | Me | NHCH₂C(O)NMe₂ | SO₂Me | H | |
| 1-112 | Me | SMe | Me | H | |
| 1-113 | Me | SOMe | Me | H | |
| 1-114 | Me | SO₂Me | Me | H | |
| 1-115 | Me | SEt | Me | H | |
| 1-116 | Me | SOEt | Me | H | |
| 1-117 | Me | SO₂Et | Me | H | |
| 1-118 | Me | S(CH₂)₂OMe | Me | H | |
| 1-119 | Me | SO(CH₂)₂OMe | Me | H | |
| 1-120 | Me | SO₂(CH₂)₂OMe | Me | H | |
| 1-121 | Me | SO₂cPr | Me | H | |
| 1-122 | Me | OH | SO₂Me | H | |
| 1-123 | Me | OMe | SO₂Me | H | |
| 1-124 | Me | OMe | SO₂Et | H | |
| 1-125 | Me | OEt | SO₂Me | H | |
| 1-126 | Me | OEt | SO₂Et | H | |
| 1-127 | Me | OiPr | SO₂Me | H | |
| 1-128 | Me | OiPr | SO₂Et | H | |
| 1-129 | Me | O(CH₂)₂OMe | SO₂Me | H | |
| 1-130 | Me | O(CH₂)₂OMe | SO₂Et | H | |
| 1-131 | Me | O(CH₂)₃OMe | SO₂Me | H | |
| 1-132 | Me | O(CH₂)₃OMe | SO₂Et | H | |
| 1-133 | Me | O(CH₂)₄OMe | SO₂Me | H | |
| 1-134 | Me | O(CH₂)₄OMe | SO₂Et | H | |
| 1-135 | Me | O(CH₂)₂NHSO2Me | SO₂Me | H | |
| 1-136 | Me | O(CH₂)₂NHSO2Me | SO₂Et | H | |
| 1-137 | Me | OCH₂(CO)NMe₂ | SO₂Me | H | |
| 1-138 | Me | OCH₂(CO)NMe₂ | SO₂Et | H | |
| 1-139 | Me | [1,4]dioxan-2-yl-methoxy | SO₂Me | H | |
| 1-140 | Me | [1,4]dioxan-2-yl-methoxy | SO₂Et | H | |
| 1-141 | Me | O(CH₂)₂-O(3,5-di-methoxypyrimidin-2-yl | SO₂Me | H | |
| 1-142 | Me | Cl | SO₂Me | H | 8.01 (d, 1H), 7.95 (s, 1H), 7.70 (d, 1 H), 7.18 (d, 1 H), 3.42 (s, 3H), 2.42 (s, 1 H) |
| 1-143 | Me | SMe | H | H | |
| 1-144 | Me | SOMe | H | H | |
| 1-145 | Me | SO₂Me | H | H | |
| 1-146 | Me | SEt | H | H | |
| 1-147 | Me | SOEt | H | H | |
| 1-148 | Me | SO₂Et | H | H | |
| 1-149 | Me | S(CH₂)₂OMe | H | H | |
| 1-150 | Me | SO(CH₂)₂OMe | H | H | |
| 1-151 | Me | SO₂(CH₂)₂OMe | H | H | |
| 1-152 | Me | SMe | F | H | |
| 1-153 | Me | SOMe | F | H | |
| 1-154 | Me | SO₂Me | F | H | |
| 1-155 | Me | SEt | F | H | |
| 1-156 | Me | SOEt | F | H | |
| 1-157 | Me | SO₂Et | F | H | |
| 1-158 | Me | S(CH₂)₂OMe | F | H | |
| 1-159 | Me | SO(CH₂)₂OMe | F | H | |
| 1-160 | Me | SO₂(CH₂)₂OMe | F | H | |
| 1-161 | Me | SMe | SO₂Me | H | |
| 1-162 | Me | SOMe | SO₂Me | H | |
| 1-163 | Me | SO₂Me | SO₂Me | H | 8.23 (d, 1 H), 8.01 (d, 1 H), 7.94 (s, 1 H), 7.19 (s, 1 H), 3.59 (s, 3H), 3.55 (s, 3H), 2.68 (s, 3H) |
| 1-164 | Me | SO₂Me | SO₂Et | H | |
| 1-165 | Me | SEt | SO₂Me | H | |
| 1-166 | Me | SOEt | SO₂Me | H | |
| 1-167 | Me | SO₂Et | SO₂Me | H | |
| 1-168 | Me | S(CH₂)₂OMe | SO₂Me | H | |
| 1-169 | Me | SO(CH₂)₂OMe | SO₂Me | H | |
| 1-170 | Me | SO₂(CH₂)₂OMe | SO2Me | H | |
| 1-171 | Me | SMe | CF₃ | H | 11.76 (s, 1 H), 7.94 (s, 1 H), 7.76 (d, 1 H), 7.66 (d, 1 H), 7.16 (s, 1 H), 2.67 (s, 3H), 2.31 (s, 3H) |
| 1-172 | Me | SOMe | CF₃ | H | 11.88 (s, 1 H), 7.94 (s, 1 H), 7.83 (d, 1 H), 7.79 (d, 1 H), 7.17 (s, 1 H), 3.04 (s, 3H), 2.83 (s, 3H) |
| 1-173 | Me | SO₂Me | CF₃ | H | |
| 1-174 | Me | SEt | CF₃ | H | |
| 1-175 | Me | SOEt | CF₃ | H | |
| 1-176 | Me | SO₂Et | CF₃ | H | |
| 1-177 | Me | S(CH₂)₂OMe | CF₃ | H | |
| 1-178 | Me | SO(CH₂)₂OMe | CF₃ | H | |
| 1-179 | Me | SO₂(CH₂)₂OMe | CF₃ | H | |
| 1-180 | Me | SMe | Br | H | |
| 1-181 | Me | SOMe | Br | H | |
| 1-182 | Me | SO₂Me | Br | H | |
| 1-183 | Me | SEt | Br | H | |
| 1-184 | Me | SOEt | Br | H | |
| 1-185 | Me | SO₂Et | Br | H | |
| 1-186 | Me | SMe | I | H | |
| 1-187 | Me | SOMe | I | H | |
| 1-188 | Me | SO₂Me | I | H | |
| 1-189 | Me | SEt | I | H | |
| 1-190 | Me | SOEt | I | H | |
| 1-191 | Me | SO₂Et | I | H | |
| 1-192 | Me | SMe | Cl | H | |
| 1-193 | Me | SOMe | Cl | H | |
| 1-194 | Me | SO₂Me | Cl | H | |
| 1-195 | Me | SEt | Cl | H | |
| 1-196 | Me | SOEt | Cl | H | |
| 1-197 | Me | SO₂Et | Cl | H | |
| 1-198 | Me | S(CH₂)₂OMe | Cl | H | |
| 1-199 | Me | SO(CH₂)₂OMe | Cl | H | |
| 1-200 | Me | SO₂(CH₂)₂OMe | Cl | H | |
| 1-201 | CH₂SMe | OMe | SO₂Me | H | |
| 1-202 | CH₂OMe | OMe | SO₂Me | H | |
| 1-203 | CH₂O(CH₂)₂ OMe | NH(CH₂)₂OEt | SO₂Me | H | |
| 1-204 | CH₂O(CH₂)₂ OMe | NH(CH₂)₃OEt | SO₂Me | H | |
| 1-205 | CH₂O(CH₂)₃ OMe | OMe | SO₂Me | H | |
| 1-206 | CH₂O(CH₂)₂ OMe | NH(CH₂)₂OMe | SO₂Me | H | |
| 1-207 | CH₂O(CH₂)₂ OMe | NH(CH₂)₃OMe | SO₂Me | H | |
| 1-208 | Et | SMe | Cl | H | |
| 1-209 | Et | SOMe | Cl | H | |
| 1-210 | Et | SO₂Me | Cl | H | |
| 1-211 | Et | SMe | CF₃ | H | |
| 1-212 | Et | SOMe | CF₃ | H | |
| 1-213 | Et | SO₂Me | CF₃ | H | |
| 1-214 | Et | F | SO₂Me | H | |
| 1-215 | Et | NH(CH₂)₂OMe | SO₂Me | H | |
| 1-216 | iPr | SMe | CF₃ | H | |
| 1-217 | iPr | SOMe | CF₃ | H | |
| 1-218 | iPr | SO₂Me | CF₃ | H | |
| 1-219 | cPr | SMe | CF₃ | H | |
| 1-220 | cPr | SOMe | CF₃ | H | |
| 1-221 | cPr | SO₂Me | CF₃ | H | |
| 1-222 | CF₃ | O(CH₂)₂OMe | F | H | |
| 1-223 | CF₃ | O(CH₂)₃OMe | F | H | |
| 1-224 | CF₃ | OCH₂CONMe₂ | F | H | |
| 1-225 | CF₃ | [1,4]dioxan-2-yl-methoxy | F | H | |
| 1-226 | CF₃ | O(CH₂)₂OMe | Cl | H | |
| 1-227 | CF₃ | O(CH₂)₃OMe | Cl | H | |
| 1-228 | CF₃ | OCH₂CONMe₂ | Cl | H | |
| 1-229 | CF₃ | [1,4]dioxan-2-yl-methoxy | Cl | H | |
| 1-230 | CF₃ | O(CH₂)₂OMe | Br | H | |
| 1-231 | CF₃ | O(CH₂)₃OMe | Br | H | |
| 1-232 | CF₃ | OCH₂CONMe₂ | Br | H | |
| 1-233 | CF₃ | [1,4]dioxan-2-yl-methoxy | Br | H | |
| 1-234 | CF₃ | O(CH₂)₂OMe | I | H | |
| 1-235 | CF₃ | O(CH₂)₃OMe | I | H | |
| 1-236 | CF₃ | OCH₂CONMe₂ | I | H | |
| 1-237 | CF₃ | [1,4]dioxan-2-yl-methoxy | I | H | |
| 1-238 | CF₃ | F | SO₂Me | H | |
| 1-239 | CF₃ | F | SO₂Et | H | |
| 1-240 | CF₃ | O(CH₂)₂OMe | SO₂Me | H | |
| 1-241 | CF₃ | O(CH₂)₂OMe | SO₂Et | H | |
| 1-242 | CF₃ | O(CH₂)₃OMe | SO₂Me | H | |
| 1-243 | CF₃ | O(CH₂)₃OMe | SO₂Et | H | |
| 1-244 | CF₃ | OCH₂CONMe₂ | SO₂Me | H | |
| 1-245 | CF₃ | OCH₂CONMe₂ | SO₂Et | H | |
| 1-246 | CF₃ | [1,4]dioxan-2-yl-methoxy | SO₂Me | H | |
| 1-247 | CF₃ | [1,4]dioxan-2-yl-methoxy | SO₂Et | H | |
| 1-248 | F | SMe | CF₃ | H | |
| 1-249 | F | SOMe | CF₃ | H | |
| 1-250 | Cl | Me | SO₂Et | H | 11.92 (s, 1 H), 8.07 (d, 1 H), 8.02 (s, 1H), 7.69 (d, 1H), 7.15 (s, 1H), 3.39 (q, 2H), 2.71 (s, 3H), 1.11 (t, 3H) |
| 1-251 | Cl | OCH₂CHCH₂ | Cl | H | |
| 1-252 | Cl | OCH₂CHF₂ | Cl | H | |
| 1-253 | Cl | O(CH₂)₂OMe | Cl | H | |
| 1-254 | Cl | OCH₂CONMe₂ | Cl | H | 11.88 (s, 1 H), 7.92 (s, 1 H), 7.69 (d, 1 H), 7.58 (d, 1 H), 7.15 (s, 1 H), 4.73 (s, 2H), 3.01 (s, 3H), 2.87 (s, 3H) |
| 1-255 | Cl | O(CH₂)-5-pyrrolidin-2-on | Cl | H | |
| 1-256 | Cl | SMe | H | H | |
| 1-257 | Cl | SOMe | H | H | |
| 1-258 | Cl | SO₂Me | H | H | |
| 1-259 | Cl | SEt | H | H | |
| 1-260 | Cl | SOEt | H | H | |
| 1-261 | Cl | SO₂Et | H | H | |
| 1-262 | Cl | S(CH₂)₂OMe | H | H | |
| 1-263 | Cl | SO(CH₂)₂OMe | H | H | |
| 1-264 | Cl | SO₂(CH₂)₂OMe | H | H | |
| 1-265 | Cl | SMe | Me | H | |
| 1-266 | Cl | SOMe | Me | H | |
| 1-267 | Cl | SO₂Me | Me | H | |
| 1-268 | Cl | SEt | Me | H | |
| 1-269 | Cl | SOEt | Me | H | |
| 1-270 | Cl | SO₂Et | Me | H | |
| 1-271 | Cl | S(CH₂)₂OMe | Me | H | |
| 1-272 | Cl | SO(CH₂)₂OMe | Me | H | |
| 1-273 | Cl | SO₂(CH₂)₂OMe | Me | H | |
| 1-274 | Cl | SMe | F | H | |
| 1-275 | Cl | SOMe | F | H | |
| 1-276 | Cl | SO₂Me | F | H | |
| 1-277 | Cl | SEt | F | H | |
| 1-278 | Cl | SOEt | F | H | |
| 1-279 | Cl | SO₂Et | F | H | |
| 1-280 | Cl | S(CH₂)₂OMe | F | H | |
| 1-281 | Cl | SO(CH₂)₂OMe | F | H | |
| 1-282 | Cl | SO₂(CH₂)₂OMe | F | H | |
| 1-283 | Cl | SMe | SO₂Me | H | 12.02 (s, 1 H), 8.09 (d, 1 H), 7.93 (s, 1 H), 7.86 (d, 1 H), 7.17 (s, 1 H), 3.57 (s, 3H), 2.52 (s, 3H) |
| 1-284 | Cl | SOMe | SO₂Me | H | 8.22 (d, 1 H), 8.10 (d, 1 H), 7.95 (s, 1 H), 7.19 (s, 1 H), 3.54 (s, 3H), 2.50 (s, 3H) |
| 1-285 | Cl | SO₂Me | SO₂Me | H | |
| 1-286 | Cl | SO₂Me | SO₂Et | H | |
| 1-287 | Cl | SEt | SO₂Me | H | |
| 1-288 | Cl | SOEt | SO₂Me | H | |
| 1-289 | Cl | SO₂Et | SO₂Me | H | |
| 1-290 | Cl | S(CH₂)₂OMe | SO₂Me | H | |
| 1-291 | Cl | SO(CH₂)₂OMe | SO₂Me | H | |
| 1-292 | Cl | SO₂(CH₂)₂OMe | SO2Me | H | |
| 1-293 | Cl | SMe | CF₃ | H | 12.01 (bs, 1H), 7.92 (s, 1H), 7.89 (d, 1 H), 7.86 (d, 1 H), 7.18 (s, 1 H), 2.09 (s, 3H) |
| 1-294 | Cl | SOMe | CF₃ | H | 12.12 (bs, 1H), 8.01-7.92 (m, 3H), 7.18 (s, 1 H), 2.50 (s, 3H) |
| 1-295 | Cl | SO₂Me | CF₃ | H | 12.20 (bs, 1H), 8.16-8.11 (m, 2H),7.96 (s, 1H), 3.52 (s, 3H), |
| 1-296 | Cl | SEt | CF₃ | H | |
| 1-297 | Cl | SOEt | CF₃ | H | |
| 1-298 | Cl | SO₂Et | CF₃ | H | |
| 1-299 | Cl | S(CH₂)₂OMe | CF₃ | H | |
| 1-300 | Cl | SO(CH₂)₂OMe | CF₃ | H | |
| 1-301 | Cl | SO₂(CH₂)₂OMe | CF₃ | H | |
| 1-302 | Cl | SMe | Br | H | |
| 1-303 | Cl | SOMe | Br | H | |
| 1-304 | Cl | SO₂Me | Br | H | |
| 1-305 | Cl | SEt | Br | H | |
| 1-306 | Cl | SOEt | Br | H | |
| 1-307 | Cl | SO₂Et | Br | H | |
| 1-308 | Cl | SMe | I | H | |
| 1-309 | Cl | SOMe | I | H | |
| 1-310 | Cl | SO₂Me | I | H | |
| 1-311 | Cl | SEt | I | H | |
| 1-312 | Cl | SOEt | I | H | |
| 1-313 | Cl | SO₂Et | I | H | |
| 1-314 | Cl | SMe | Cl | H | |
| 1-315 | Cl | SOMe | Cl | H | |
| 1-316 | Cl | SO₂Me | Cl | H | |
| 1-317 | Cl | SEt | Cl | H | |
| 1-318 | Cl | SOEt | Cl | H | |
| 1-319 | Cl | SO₂Et | Cl | H | |
| 1-320 | Cl | S(CH₂)₂OMe | Cl | H | |
| 1-321 | Cl | SO(CH₂)₂OMe | Cl | H | |
| 1-322 | Cl | SO₂(CH₂)₂OMe | Cl | H | |
| 1-323 | Cl | F | SMe | H | |
| 1-324 | Cl | Cl | SO₂Me | H | |
| 1-325 | Cl | COOMe | SO₂Me | H | |
| 1-326 | Cl | CONMe₂ | SO₂Me | H | |
| 1-327 | Cl | CONMe(OMe) | SO₂Me | H | |
| 1-328 | Cl | CH₂OMe | SO₂Me | H | |
| 1-329 | Cl | CH₂OMe | SO₂Et | H | |
| 1-330 | Cl | CH₂OEt | SO₂Me | H | |
| 1-331 | Cl | CH₂OEt | SO₂Et | H | |
| 1-332 | Cl | CH₂OCH₂CH₂OMe | SO₂Me | H | |
| 1-333 | Cl | CH₂OCH₂CHF₂ | SO₂Me | H | |
| 1-334 | Cl | CH₂OCH₂CF₃ | SO₂Me | H | 12.05 (bs, 1H), 8.06 (d, 1H), 7.94-7.89 (m, 2H), 7.18 (s, 1H), 5.24 (s, 2H), 4.28 (q, 2H), 3.35 (s, 3H) |
| 1-335 | Cl | CH₂OCH₂CF₃ | SO₂Et | H | |
| 1-336 | Cl | CH₂OCH₂CF₂CHF₂ | SO₂Me | H | |
| 1-337 | Cl | CH₂OcPentyl | SO₂Me | H | |
| 1-338 | Cl | CH₂PO(OMe)₂ | SO₂Me | H | |
| 1-339 | Cl | 4,5-dihydro-1,2-oxazol-3 yl | SMe | H | |
| 1-340 | Cl | 4,5-dihydro-1,2-oxazol-3 yl | SO₂Me | H | |
| 1-341 | Cl | 4,5-dihydro-1,2-oxazol-3 yl | SO₂Et | H | |
| 1-342 | Cl | 5-cyanomethyl- 4,5-dihydro-1,2-oxazol-3 yl | SO₂Me | H | |
| 1-343 | Cl | 5-cyanomethyl- 4,5-dihydro-1,2-oxazol-3 yl | SO₂Et | H | |
| 1-344 | Cl | 5-(Methoxymethyl)-4,5-dihydro-1,2-oxazol-3 yl | SO₂Et | H | |
| 1-345 | Cl | 5-(Methoxymethyl)-5-methyl- 4,5-dihydro-1,2-oxazol-3 yl | SO₂Et | H | |
| 1-346 | Cl | CH₂O-tetrahydrofuran-3-yl | SO₂Me | H | |
| 1-347 | Cl | CH₂O-tetra-hydrofuran-3-yl | SO₂Et | H | |
| 1-348 | Cl | CH₂OCH₂-tetrahydrofuran-2-yl | SO₂Me | H | |
| 1-349 | Cl | CH₂OCH₂-tetra-hydrofuran-2-yl | SO₂Et | H | |
| 1-350 | Cl | CH₂OCH₂-tetra-hydrofuran-3-yl | SO₂Me | H | |
| 1-351 | Cl | CH₂OCH₂-tetra-hydrofuran-3-yl | SO₂Et | H | |
| 1-352 | Cl | OMe | SO₂Me | H | |
| 1-353 | Cl | OMe | SO₂Et | H | |
| 1-354 | Cl | OEt | SO₂Me | H | |
| 1-355 | Cl | OEt | SO₂Et | H | |
| 1-356 | Cl | OiPr | SO₂Me | H | |
| 1-357 | Cl | OiPr | SO₂Et | H | |
| 1-358 | Cl | OnPr | SO₂Me | H | |
| 1-359 | Cl | O(CH₂)₂F | SO₂Me | H | |
| 1-360 | Cl | O(CH₂)₂OMe | SO₂Me | H | |
| 1-361 | Cl | O(CH₂)₄OMe | SO₂Me | H | |
| 1-362 | Cl | O(CH₂)₄OMe | SO₂Et | H | |
| 1-363 | Cl | O(CH₂)₃OMe | SO₂Me | H | |
| 1-364 | Cl | O(CH₂)₃OMe | SO₂Et | H | |
| 1-365 | Cl | O(CH₂)₂OMe | SO₂Me | H | |
| 1-366 | Cl | O(CH₂)₂OMe | SO₂Et | H | |
| 1-367 | Cl | OCH₂-c-Pr | SO₂Et | H | |
| 1-368 | Cl | [1,4]dioxan-2-yl-methoxy | SO₂Me | H | |
| 1-369 | Cl | [1,4]dioxan-2-yl-methoxy | SO₂Et | H | |
| 1-370 | Cl | OCH₂(CO)NMe₂ | SO₂Me | H | |
| 1-371 | Cl | OCH₂(CO)NMe₂ | SO₂Et | H | |
| 1-372 | Br | OMe | Br | H | |
| 1-373 | Br | O(CH₂)₂OMe | Br | H | |
| 1-374 | Br | O(CH₂)₂OMe | SO₂Me | H | |
| 1-375 | Br | O(CH₂)₂OMe | SO₂Et | H | |
| 1-376 | Br | O(CH₂)₃OMe | SO₂Me | H | |
| 1-377 | Br | O(CH₂)₃OMe | SO₂Et | H | |
| 1-378 | Br | O(CH₂)₄OMe | SO₂Me | H | |
| 1-379 | Br | O(CH₂)₄OMe | SO₂Et | H | |
| 1-380 | Br | [1,4]dioxan-2-yl-methoxy | SO₂Me | H | |
| 1-381 | Br | [1,4]dioxan-2-yl-methoxy | SO₂Et | H | |
| 1-382 | I | O(CH₂)₂OMe | SO₂Me | H | |
| 1-383 | I | O(CH₂)₂OMe | SO₂Et | H | |
| 1-384 | I | O(CH₂)₃OMe | SO₂Me | H | |
| 1-385 | I | O(CH₂)₃OMe | SO₂Et | H | |
| 1-386 | I | O(CH₂)₄OMe | SO₂Me | H | |
| 1-387 | I | O(CH₂)₄OMe | SO₂Et | H | |
| 1-388 | I | [1,4]dioxan-2-yl-methoxy | SO₂Me | H | |
| 1-389 | I | [1,4]dioxan-2-yl-methoxy | SO₂Et | H | |
| 1-390 | OMe | SMe | CF₃ | H | |
| 1-391 | OMe | SOMe | CF₃ | H | |
| 1-392 | OMe | SO₂Me | CF₃ | H | |
| 1-393 | OMe | SEt | CF₃ | H | |
| 1-394 | OMe | SOEt | CF₃ | H | |
| 1-395 | OMe | SO₂Et | CF₃ | H | |
| 1-396 | OMe | S(CH₂)₂OMe | CF₃ | H | |
| 1-397 | OMe | SO(CH₂)₂OMe | CF₃ | H | |
| 1-398 | OMe | SO₂(CH₂)₂OMe | CF₃ | H | |
| 1-399 | OMe | SMe | CHF₂ | H | |
| 1-400 | OMe | SOMe | CHF₂ | H | |
| 1-401 | OMe | SO₂Me | CHF₂ | H | |
| 1-402 | OMe | SEt | CHF₂ | H | |
| 1-403 | OMe | SOEt | CHF₂ | H | |
| 1-404 | OMe | SO₂Et | CHF₂ | H | |
| 1-405 | OMe | S(CH₂)₂OMe | CHF₂ | H | |
| 1-406 | OMe | SO(CH₂)₂OMe | CHF₂ | H | |
| 1-407 | OMe | SO₂(CH₂)₂OMe | CHF₂ | H | |
| 1-408 | OMe | SMe | Cl | H | |
| 1-409 | OMe | SOMe | Cl | H | |
| 1-410 | OMe | SO₂Me | Cl | H | |
| 1-411 | OMe | SEt | Cl | H | |
| 1-412 | OMe | SOEt | Cl | H | |
| 1-413 | OMe | SO2Et | Cl | H | |
| 1-414 | OMe | S(CH₂)₂OMe | Cl | H | |
| 1-415 | OMe | SO(CH₂)₂OMe | Cl | H | |
| 1-416 | OMe | SO₂(CH₂)₂OMe | Cl | H | |
| 1-417 | OEt | SMe | CF₃ | H | |
| 1-418 | OEt | SOMe | CF₃ | H | |
| 1-419 | OEt | SO₂Me | CF₃ | H | |
| 1-420 | OEt | SEt | CF₃ | H | |
| 1-421 | OEt | SOEt | CF₃ | H | |
| 1-422 | OEt | SO₂Et | CF₃ | H | |
| 1-423 | OEt | S(CH₂)₂OMe | CF₃ | H | |
| 1-424 | OEt | SO(CH₂)₂OMe | CF₃ | H | |
| 1-425 | OEt | SO₂(CH₂)₂OMe | CF₃ | H | |
| 1-426 | OEt | SMe | CHF₂ | H | |
| 1-427 | OEt | SOMe | CHF₂ | H | |
| 1-428 | OEt | SO₂Me | CHF₂ | H | |
| 1-429 | OEt | SEt | CHF₂ | H | |
| 1-430 | OEt | SOEt | CHF₂ | H | |
| 1-431 | OEt | SO₂Et | CHF₂ | H | |
| 1-432 | OEt | S(CH₂)₂OMe | CHF₂ | H | |
| 1-433 | OEt | SO(CH₂)₂OMe | CHF₂ | H | |
| 1-434 | OEt | SO₂(CH₂)₂OMe | CHF₂ | H | |
| 1-435 | OEt | SMe | Cl | H | |
| 1-436 | OEt | SOMe | Cl | H | |
| 1-437 | OEt | SO₂Me | Cl | H | |
| 1-438 | OEt | SEt | Cl | H | |
| 1-439 | OEt | SOEt | Cl | H | |
| 1-440 | OEt | SO2Et | Cl | H | |
| 1-441 | OEt | S(CH₂)₂OMe | Cl | H | |
| 1-442 | OEt | SO(CH₂)₂OMe | Cl | H | |
| 1-443 | OEt | SO₂(CH₂)₂OMe | Cl | H | |
| 1-444 | O(CH₂)c-Pr | SMe | CF₃ | H | |
| 1-445 | O(CH₂)c-Pr | SOMe | CF₃ | H | |
| 1-446 | O(CH₂)c-Pr | SO₂Me | CF₃ | H | |
| 1-447 | O(CH₂)c-Pr | SEt | CF₃ | H | |
| 1-448 | O(CH₂)c-Pr | SOEt | CF₃ | H | |
| 1-449 | O(CH₂)c-Pr | SO₂Et | CF₃ | H | |
| 1-450 | O(CH₂)c-Pr | S(CH₂)₂OMe | CF₃ | H | |
| 1-451 | O(CH₂)c-Pr | SO(CH₂)₂OMe | CF₃ | H | |
| 1-452 | O(CH₂)c-Pr | SO₂(CH₂)₂OMe | CF₃ | H | |
| 1-453 | O(CH₂)c-Pr | SMe | Cl | H | |
| 1-454 | O(CH₂)c-Pr | SOMe | Cl | H | |
| 1-455 | O(CH₂)c-Pr | SO₂Me | Cl | H | |
| 1-456 | O(CH₂)c-Pr | SEt | Cl | H | |
| 1-457 | O(CH₂)c-Pr | SOEt | Cl | H | |
| 1-458 | O(CH₂)c-Pr | SO₂Et | Cl | H | |
| 1-459 | O(CH₂)c-Pr | S(CH₂)₂OMe | Cl | H | |
| 1-460 | O(CH₂)c-Pr | SO(CH₂)₂OMe | Cl | H | |
| 1-461 | O(CH₂)c-Pr | SO₂(CH₂)₂OMe | Cl | H | |
| 1-462 | O(CH₂)c-Pr | SMe | SO₂Me | H | |
| 1-463 | O(CH₂)c-Pr | SOMe | SO₂Me | H | |
| 1-464 | O(CH₂)c-Pr | SO₂Me | SO₂Me | H | |
| 1-465 | O(CH₂)c-Pr | SEt | SO₂Me | H | |
| 1-466 | O(CH₂)c-Pr | SOEt | SO₂Me | H | |
| 1-467 | O(CH₂)c-Pr | SO₂Et | SO₂Me | H | |
| 1-468 | O(CH₂)c-Pr | S(CH₂)₂OMe | SO₂Me | H | |
| 1-469 | O(CH₂)c-Pr | SO(CH₂)₂OMe | SO₂Me | H | |
| 1-470 | O(CH₂)c-Pr | SO₂(CH₂)₂OMe | SO₂Me | H | |
| 1-471 | SO₂Me | F | CF₃ | H | |
| 1-472 | SO₂Me | NH₂ | CF₃ | H | |
| 1-473 | SO₂Me | NHEt | Cl | H | |
| 1-474 | SMe | SEt | F | H | |
| 1-475 | SMe | SMe | F | H | |
| 1-476 | Me | NH₂ | Cl | H | |
| 1-477 | Me | NHMe | Cl | H | |
| 1-478 | Me | NMe₂ | Cl | H | |
| 1-479 | Me | pyrazol-1-yl | Cl | H | |
| 1-480 | Me | NH₂ | Br | H | |
| 1-481 | Me | NHMe | Br | H | |
| 1-482 | Me | NMe₂ | Br | H | |
| 1-483 | Me | NH₂ | SO₂Me | H | |
| 1-484 | Me | NHMe | SO₂Me | H | |
| 1-485 | Me | NMe₂ | SO₂Me | H | |
| 1-486 | Me | NH(CH₂)₂OMe | SO₂Me | H | |
| 1-487 | Me | morpholin-4-yl | SO₂Me | H | |
| 1-488 | Me | 1,2,3-triazol-1-yl | SO₂Me | H | |
| 1-489 | Me | 1,2,3-triazol-2-yl | SO₂Me | H | |
| 1-490 | Me | pyrazol-1-yl | SO₂Me | H | |
| 1-491 | Me | 1,2,4-triazol-1-yl | SO₂Me | H | |
| 1-492 | Me | NH₂ | CF₃ | H | |
| 1-493 | Me | NHMe | CF₃ | H | |
| 1-494 | Me | NMe₂ | CF₃ | H | |
| 1-495 | Me | NH(CH₂)₂OMe | CF₃ | H | |
| 1-496 | Me | morpholin-4-yl | CF₃ | H | |
| 1-497 | Me | 1,2,3-triazol-1-yl | CF₃ | H | |
| 1-498 | Me | 1,2,3-triazol-2-yl | CF₃ | H | |
| 1-499 | Me | pyrazol-1-yl | CF₃ | H | |
| 1-500 | Me | 1,2,4-triazol-1-yl | CF₃ | H | |
| 1-501 | Cl | NH₂ | Cl | H | |
| 1-502 | Cl | NHMe | Cl | H | |
| 1-503 | Cl | NMe₂ | Cl | H | |
| 1-504 | Cl | pyrazol-1-yl | Cl | H | |
| 1-505 | Cl | NH₂ | Br | H | |
| 1-506 | Cl | NHMe | Br | H | |
| 1-507 | Cl | NMe₂ | Br | H | |
| 1-508 | Cl | NH₂ | SO₂Me | H | |
| 1-509 | Cl | NHMe | SO₂Me | H | |
| 1-510 | Cl | NMe₂ | SO₂Me | H | |
| 1-511 | Cl | NH(CH₂)₂OMe | SO₂Me | H | |
| 1-512 | Cl | morpholin-4-yl | SO₂Me | H | |
| 1-513 | Cl | 1,2,3-triazol-1-yl | SO₂Me | H | |
| 1-514 | Cl | 1,2,3-triazol-2-yl | SO₂Me | H | |
| 1-515 | Cl | pyrazol-1-yl | SO₂Me | H | |
| 1-516 | Cl | 1,2,4-triazol-1-yl | SO₂Me | H | |
| 1-517 | Cl | NH₂ | CF₃ | H | |
| 1-518 | Cl | NHMe | CF₃ | H | |
| 1-519 | Cl | NMe₂ | CF₃ | H | |
| 1-520 | Cl | NH(CH₂)₂OMe | CF₃ | H | |
| 1-521 | Cl | morpholin-4-yl | CF₃ | H | |
| 1-522 | Cl | 1,2,3-triazol-1-yl | CF₃ | H | |
| 1-523 | Cl | 1,2,3-triazol-2-yl | CF₃ | H | |
| 1-524 | Cl | pyrazol-1-yl | CF₃ | H | |
| 1-525 | Cl | 1,2,4-triazol-1-yl | CF₃ | H | |
| 1-526 | Cl | Sulfoximin 1 | Cl | H | |
| 1-527 | Cl | Sulfoximin 2 | Cl | H | |
| 1-528 | Cl | Sulfoximin 3 | Cl | H | |
| 1-529 | Cl | Sulfoximin 4 | Cl | H | |
| 1-530 | Cl | Sulfoximin 5 | Cl | H | |
| 1-531 | Cl | Sulfoximin 3 | OMe | H | |
| 1-532 | Cl | Sulfoximin 4 | OMe | H | |
| 1-533 | Cl | Sulfoximin 5 | OMe | H | |
| 1-534 | Cl | Sulfoximin 3 | COOMe | H | |
| 1-535 | Cl | Sulfoximin 4 | COOMe | H | |
| 1-536 | Cl | Sulfoximin 5 | COOMe | H | |
| 1-537 | OMe | Sulfoximin 3 | OMe | H | |
| 1-538 | OMe | Sulfoximin 4 | OMe | H | |
| 1-539 | OMe | Sulfoximin 5 | OMe | H | |
| 1-540 | Me | Sulfoximin 1 | CF₃ | H | |
| 1-541 | Me | Sulfoximin 2 | CF₃ | H | |
| 1-542 | Me | Sulfoximin 1 | SO₂Me | H | |
| 1-543 | Me | Sulfoximin 2 | SO₂Me | H | |
| 1-544 | Me | Sulfoximin 1 | Cl | H | |
| 1-545 | Me | Sulfoximin 2 | Cl | H | |

**Tabelle 2: Erfindungsgemäße Verbindungen der allgemeinen Formel (I), worin R für Methyl und R' für Wasserstoff, A für C-Y steht, und V, X, Y sowie Z die in Tabelle 1 angegebenen Bedeutungen haben.**

| | | | | | |
|---|---|---|---|---|---|
| 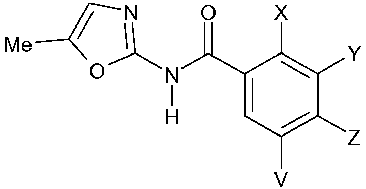 | | | | | |

| Nr. | X | Y | Z | V | Physikalische Daten (¹H-NMR, DMSO-d₆, 400 MHz) |
|---|---|---|---|---|---|
| 2-173 | Me | SO₂Me | CF₃ | H | 11.82 (s, 1 H), 8.00 (d, 1 H), 7.93 (d, 1 H), 7.63 (s, 1 H), 3.41 (s, 3H), 2.70 (s, 3H), 2.07 (s, 3H) |

Tabelle 3: Erfindungsgemäße Verbindungen der allgemeinen Formel (I), worin R für Ethyl und R'für Wasserstoff, A für C-Y steht, und V, X, Y sowie Z die in Tabelle 1 angegebenen Bedeutungen haben.

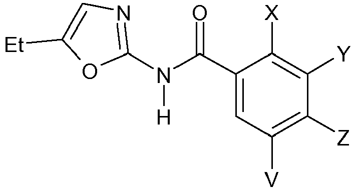

Tabelle 4: Erfindungsgemäße Verbindungen der allgemeinen Formel (I), worin R für Phenyl und R'für Wasserstoff, A für C-Y steht, und V, X, Y sowie Z die in Tabelle 1 angegebenen Bedeutungen haben.

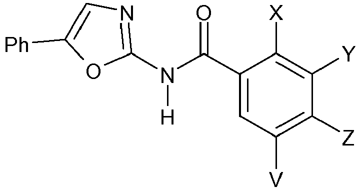

**Tabelle 5: Erfindungsgemäße Verbindungen der allgemeinen Formel (I), worin R für Benzyl und R'für Wasserstoff, A für C-Y steht, und V, X, Y sowie Z die in Tabelle 1 angegebenen Bedeutungen haben.**

| | | | | | |
|---|---|---|---|---|---|
| 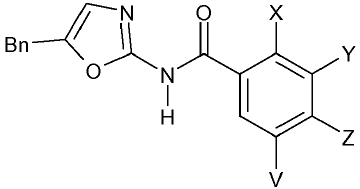 | | | | | |

| Nr. | X | Y | Z | V | Physikalische Daten (¹H-NMR, DMSO-d₆, 400 MHz) |
|---|---|---|---|---|---|
| 5-163 | Me | SO₂Me | SO₂Me | H | 11.81 (s, 1H), 8.19 (d, 1 H), 7.97 (d, 1H), 7.34-7.25 (m, 5H), 6.66 (s, 1H), 4.02 (s, 2H), 3.58 (s, 3H), 3.54 (s, 3H), 2.66 (s, 3H) |
| 5-173 | Me | SO₂Me | CF₃ | H | 11.79 (bs, 1H), 8.01 (d, 1H), 7.97 (d, 1H), 7.34-7.25 (m, 5H), 6.64 (s, 1H), 4.02 (s, 2H), 3.39 (s, 3H), 2.71 (s, 3H) |

**Tabelle 6: Erfindungsgemäße Verbindungen der allgemeinen Formel (I), worin R für Trifluormethyl und R'für Wasserstoff, A für C-Y steht, und V, X, Y sowie Z die in Tabelle 1 angegebenen Bedeutungen haben.**

| | | | | | |
|---|---|---|---|---|---|
| 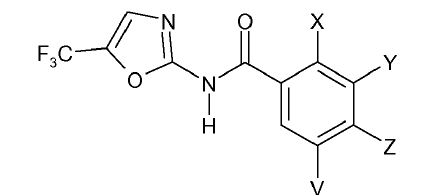 | | | | | |

| Nr. | X | Y | Z | V | Physikalische Daten (¹H-NMR, DMSO-d₆, 400 MHz) |
|---|---|---|---|---|---|
| 6-173 | Me | SO₂Me | CF₃ | H | 7.92 (d, 1 H), 7.85 (d, 1 H), 7.26 (s, 1 H), 3.26 (s, 3H), 2.87 (s, 3H), |

**Tabelle 7: Erfindungsgemäße Verbindungen der allgemeinen Formel (I), worin R für Wasserstoff und R'für Methyl, A für C-Y steht, und V, X, Y sowie Z die in Tabelle 1 angegebenen Bedeutungen haben.**

| | | | | | |
|---|---|---|---|---|---|
| 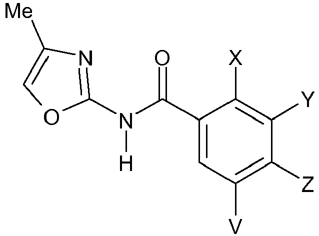 | | | | | |

| Nr. | X | Y | Z | V | Physikalische Daten (¹H-NMR, DMSO-d₆, 400 MHz) |
|---|---|---|---|---|---|
| 7-13 | Cl | H | SO₂Me | H | 11.89 (s, 1H), 8.11 (s, 1H), 7.99 (d, 1 H), 7.88 (d, 1 H), 7.61 (d, 1 H) 3.33 (s, 3H), 2.07 (s, 3H) |
| 7-334 | Cl | CH₂OCH₂CF₃ | SO₂Me | H | 12.94 (s, 1 H), 8.09 (d, 1 H), 7.88 (d, 1 H), 7.62 (s, 1 H), 5.24 (s, 2H), 4.28 (q, 2H), 3.36 (s, 3H), 2.08 (s, 3H) |

**Tabelle 8: Erfindungsgemäße Verbindungen der allgemeinen Formel (I), worin R für Wasserstoff, R'für Trifluormethyl und A für C-Y steht, und V, X, Y sowie Z die in Tabelle 1 angegebenen Bedeutungen haben.**

| | | | | | |
|---|---|---|---|---|---|
| 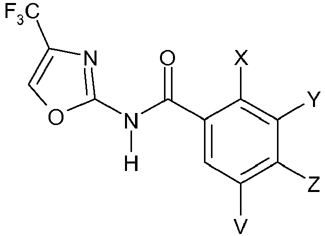 | | | | | |

| Nr. | X | Y | Z | V | Physikalische Daten (¹H-NMR, DMSO-d₆, 400 MHz) |
|---|---|---|---|---|---|
| 8-13 | Cl | H | SO₂Me | H | 12.52 (s, 1 H), 8.75 (s, 1 H), 8.13 (d, 1 H), 8.01 (d, 1 H), 7.95 (d, 1 H) 3.33 (s, 3H) |
| 8-29 | CF₃ | H | SO₂Me | H | 12.65 (s, 1 H), 8.75 (s, 1 H), 8.37-8.22 (m, 2H), 8.08 (d, 1H), 3.34 (s, 3H) |
| 8-32 | NO₂ | H | NO₂ | H | 12.75 (s, 1H), 8.89 (s, 1H), 8.81-8.65 (m, 2H), 8.13 (d, 1H), |
| 8-37 | NO₂ | H | F | H | 12.79 (s, 1H), 8.89 (s, 1H), 8.80-8.67 (m, 2H), 8.13 (d, 1 H), |
| 8-38 | NO₂ | H | Cl | H | 12.51 (s, 1 H), 8.71 (s, 1 H), 8.31 (d, 1 H), 8.01 (dd, 1 H), 7.87 (d, 1 H) |
| 8-39 | NO₂ | H | Br | H | 12.50 (s, 1 H), 8.71 (s, 1 H), 8.41 (d, 1 H), 8.14 (dd, 1H), 7.78 (d, 1H) |
| 8-42 | NO₂ | H | SO₂Me | H | 12.67 (s, 1 H), 8.74 (s, 1 H), 8.66 (d, 1 H), 8.44 (dd, 1H), 8.12(d, 1H), 3.42 (s, 3H) |
| 8-44 | NO₂ | H | CF₃ | H | 12.62 (s, 1 H), 8.72 (s, 1 H), 8.55 (s, 1 H), 7.33 (d, 1 H), 8.06 (d, 1 H) |
| 8-172 | Me | SOMe | CF₃ | H | 13.38 (s, 1H), 8.73 (s, 1H), 7.87-7.82 (m, 2H), 3.06 (s, 3H), 2.83 (s, 3H) |
| 8-173 | Me | SO₂Me | CF₃ | H | 12.43 (s, 1 H), 8.73 (s, 1 H), 8.03 (d, 1 H), 7.98 (d, 1 H), 3.42 (s, 3H), 2.72 (s, 3H) |
| 8-249 | F | SOMe | CF₃ | H | |
| 8-334 | Cl | CH₂OCH₂CF₃ | SO₂Me | H | 12.60 (s, 1H), 8.74 (s, 1H), 8.11 (d, 1 H), 7.95 (d, 1H), 5.25 (s, 2H), 4.30 (q, 2H), 3.36 (s, 3H) |
| 8-343 | Cl | 5-cyanomethyl-4,5-dihydro-1,2-oxazol-3 yl | SO₂Et | H | 12.60 (s, 1 H), 8.73 (s, 1 H), 8.11 (d, 1 H), 8.07 (d, 1 H), 5.19 (m, 1 H), 3.66-3.54 (m, 1 H), 3.25-3.49 (m, 5H), 3.17 (dd, 1 H), 3.09-2.95 (m, 2H), 1.15 (t, 3H) |
| 8-348 | Cl | CH₂OCH₂-tetrahydrofuran-2-yl | SO₂Me | | 12.54 (s, 1 H), 8.73 (s, 1 H), 8.00 (d,1H), 7.89 (d, 1H), 5.08 (s, 2H), 3.97 (m, 1H), 3.70 (dd, 1 H), 3.65-3.51 (m, 3H), 3.40 (s, 3H), 1.93-1.70 (m, 3H), 1.58-1.49 (m, 1 H). |

**Tabelle 9: Erfindungsgemäße Verbindungen der allgemeinen Formel (I), worin R für Wasserstoff, R'für Phenyl und A für C-Y steht, und V, X, Y sowie Z die in Tabelle 1 angegebenen Bedeutungen haben.**

| | | | | | |
|---|---|---|---|---|---|
| 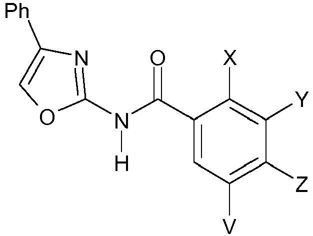 | | | | | |

| **Nr.** | **X** | **Y** | **Z** | **V** | **Physikalische Daten** (¹H-NMR, DMSO-d₆, 400 MHz) |
|---|---|---|---|---|---|
| 9-13 | Cl | H | SO₂Me | H | 8.87 (s, 1 H), 8.11 (s, 1 H), 8.04 (d, 1 H), 8.00 (d, 1 H), 7.87 (m, 2H), 7.49 (m, 3H), 6.92 (s, 1 H), 3.12 (s, 3H) |
| 9-334 | Cl | CH₂OCH₂CF₃ | SO₂Me | H | 8.99 (s, 1 H), 8.17 (d, 1 H), 7.89-7.80 (m, 3H), 7.49 (m, 3H), 6.91 (s, 1H), 5.38 (s, 2H), 4.08 (q, 2H), 3.20 (s, 3H) |

**Tabelle 10: Erfindungsgemäße Verbindungen der allgemeinen Formel (I), worin A für N steht.**

| | | | | | | |
|---|---|---|---|---|---|---|
| 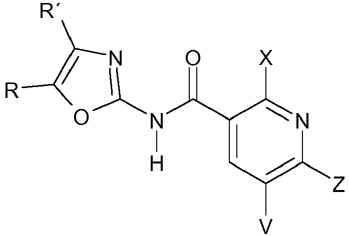 | | | | | | |

| **Nr.** | **R** | **R'** | **X** | **Z** | **V** | **Physikalische Daten** (¹H-NMR, DMSO-d₆, 400 MHz) |
|---|---|---|---|---|---|---|
| 10-1 | H | H | Cl | CF₃ | H | 8.34 (d, 1 H), 7.71 (d, 1 H), 7.41 (s, 1H), 6.92 (s, 1H) |
| 10-2 | H | H | Me | CF₃ | H | 8.22 (d, 1 H), 7.94 (s, 1 H), 7.86 (d, 1H), 7.18 (s, 1 H, 2.65 (s, 3H)) |
| 10-3 | H | H | CH₂OMe | CF₃ | H | |
| 10-4 | | | | | | |
| 10-5 | H | H | CH₂OCH₂CH₂OMe | CF₃ | H | |
| 10-6 | H | H | | | | |
| 10-7 | H | Me | Cl | CF₃ | H | |
| 10-8 | H | Me | Me | CF₃ | H | |
| 10-9 | H | Me | CH₂OMe | CF₃ | H | |
| 10-10 | | | | | | |
| 10-11 | H | Me | CH₂OCH₂CH₂OMe | CF₃ | H | |
| 10-12 | H | Me | | | | |
| 10-13 | H | Et | Cl | CF₃ | H | |
| 10-14 | H | Et | Me | CF₃ | H | |
| 10-15 | H | Et | CH₂OMe | CF₃ | H | |
| 10-16 | | | | | | |
| 10-17 | H | Et | CH₂OCH₂CH₂OMe | CF₃ | H | |
| 10-18 | H | Et | | | | |
| 10-19 | H | CF₃ | Cl | CF₃ | H | |
| 10-20 | H | CF₃ | Me | CF₃ | H | |
| 10-21 | H | CF₃ | CH₂OMe | CF₃ | H | |
| 10-22 | | | | | | |
| 10-23 | H | CF₃ | CH₂OCH₂CH₂OMe | CF₃ | H | |
| 10-24 | H | CF₃ | | | | |
| 10-25 | H | Ph | Cl | CF₃ | H | 9.32 (bs, 1 H), 8.43 (d, 1 H), 7.88-7.69 (m, 3H), 7.51-7.47 (m, 3H), 6.90 (s, 1 H) |
| 10-26 | H | Ph | Me | CF₃ | H | |
| 10-27 | H | Ph | CH₂OMe | CF₃ | H | |
| 10-28 | | | | | | |
| 10-29 | H | Ph | CH₂OCH₂CH₂OMe | CF₃ | H | |
| 10-30 | H | Ph | | | | |
| 10-31 | Me | H | Cl | CF₃ | H | |
| 10-32 | Me | H | Me | CF₃ | H | |
| 10-33 | Me | H | CH₂OMe | CF₃ | H | |
| 10-34 | | | | | | |
| 10-35 | Me | H | CH₂OCH₂CH₂OMe | CF₃ | H | |
| 10-36 | Me | H | | | | |
| 10-37 | Et | H | Cl | CF₃ | H | |
| 10-38 | Et | H | Me | CF₃ | H | |
| 10-39 | Et | H | CH₂OMe | CF₃ | H | |
| 10-40 | | | | | | |
| 10-41 | Et | H | CH₂OCH₂CH₂OMe | CF₃ | H | |
| 10-42 | Et | H | | | | |
| 10-43 | CF₃ | H | Cl | CF₃ | H | |
| 10-44 | CF₃ | H | Me | CF₃ | H | |
| 10-45 | CF₃ | H | CH₂OMe | CF₃ | H | |
| 10-46 | | | | | | |
| 10-47 | CF₃ | H | CH₂OCH₂CH₂OMe | CF₃ | H | |
| 10-48 | CF₃ | H | | | | |
| 10-49 | Ph | H | Cl | CF₃ | H | |
| 10-50 | Ph | H | Me | CF₃ | H | |
| 10-51 | Ph | H | CH₂OMe | CF₃ | H | |
| 10-52 | | | | | | |
| 10-53 | Ph | H | CH₂OCH₂CH₂OMe | CF₃ | H | |
| 10-54 | Ph | H | | | | |
| 10-55 | Me | Me | Cl | CF₃ | H | 8.48 (d, 1 H), 7.64 (d, 1 H), 2.22 (s, 3H), 2.12 (s, 3H) |
| 10-56 | Me | Me | Me | CF₃ | H | |
| 10-57 | Me | Me | CH₂OMe | CF₃ | H | |
| 10-58 | | | | | | |
| 10-59 | Me | Me | CH₂OCH₂CH₂OMe | CF₃ | H | |
| 10-60 | Me | Me | | | | |
| 10-61 | Ph | Ph | Cl | CF₃ | H | |
| 10-62 | Ph | Ph | Me | CF₃ | H | |
| 10-63 | Ph | Ph | CH₂OMe | CF₃ | H | |
| 10-64 | | | | | | |
| 10-65 | Ph | Ph | CH₂OCH₂CH₂OMe | CF₃ | H | |
| 10-66 | Ph | Ph | | | | |

**Tabelle 11: Erfindungsgemäße Verbindungen der allgemeinen Formel (I), worin R und R' für Methyl stehen und A für C-Y steht und V, X, Y sowie Z die in Tabelle 1 angegebenen Bedeutungen haben.**

| | | | | | |
|---|---|---|---|---|---|
| 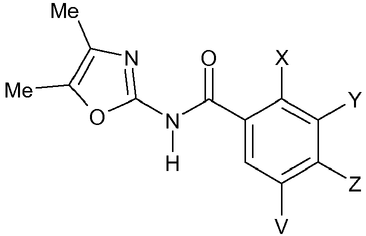 | | | | | |

| Nr. | X | Y | Z | V | Physikalische Daten (¹H-NMR, DMSO-d₆, 400 MHz) |
|---|---|---|---|---|---|
| 11-13 | Cl | H | SO₂Me | H | 11.48 (s, 1 H), 8.02-7.97 (m, 2H), 7.86 (d, 1H), 3.07 (s, 3H), 2.24 (s, 3H), 2.11 (s, 3H) |
| 11-29 | SO₂Me | | CF₃ | H | 11.89(s, 1H), 8.30-8.14 (m, 2H), 7.92 (d, 1H), 3.50 (s, 3H), 2.20 (s, 3H), 2.02 (s, 3H) |
| 11-173 | Cl | SO₂Me | CF₃ | H | 7.98 (d, 1H), 7.90 (d, 1H), 3.37 (s, 3H), 2.70 (s, 3H), 2.22 (s, 3H), 2.01 (s, 3H) |
| 11-334 | Cl | CH₂OCH₂CF₃ | SO₂Me | H | 8.11 (d, 1H), 7.83 (d, 1H), 5.39 (s, 2H), 4.02 (q, 2H), 3.20 (s, 3H), 2.24 (s, 3H), 2.13 (s, 3H) |
| 11-343 | Cl | 5-cyanomethyl-4,5-dihydro-1,2-oxazol-3 yl | SO₂Et | H | 8.11 (d, 1 H), 8.07 (d, 1H), 5.18 (m, 1 H), 3.62-3.54 (m, 1 H), 3.25-3.49 (m, 2H), 3.17 (dd, 1H), 3.09-2.96 (m, 2H), 2.21 (s, 3H), 2.01 (s, 3H), 1.15 (t, 3H) |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-163, 1-171, 1-172, 1-283, 1-284, 1-294, 1-295, 1-334, 10-001 und 11-343 bei einer Aufwandmenge von 320 g/ha eine mindestens 80%-ige Wirkung gegen gegen Veronica persica.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigt beispielsweise die Verbindung Nr. 1-163, 1-171, 1-172, 1-173, 1-254, 1-283, 1-284, 1-293, 1-294 und 1-334 bei einer Aufwandmenge von 80 g/ha eine mindestens 80%-ige Wirkung gegen Abutilon theophrasti und Amaranthus retroflexus.

### 3. Vergleichsversuche

In den nachfolgenden Tabellen werden die Eigenschaften von erfindungsgemäßen mit den strukturell nächstliegenden und aus WO 2012/028579 A1 bekannten Verbindungen verglichen. Diese Versuche wurden unten den unter Punkt 1 und 2 genannten Bedingungen im Vor- und Nachauflauf durchgeführt. Dabei wurde die herbizide Wirkung gegen unterschiedliche Schadpflanzen ebenso wie die Schädigung von einigen bedeutenden Kulturpflanzen bei verschiedenen Dosierungen verglichen.

Die hier verwendeten Abkürzungen bedeuten:

**Schadpflanzen**

| | | | |
|---|---|---|---|
| ABUTH | Abutilon theophrasti | ALOMY | Alopecurus myosuroides |
| AMARE | Amaranthus retroflexus | AVEFA | Avena fatua |
| CYPES | Cyperus serotinus | ECHCG | Echinochloa crus galli |
| LOLMU | Lolium multiflorum | MATIN | Matricaria inodora |
| PHBPU | Pharbitis purpureum | POLCO | Polygonum convolvulus |
| SETVI | Setaria viridis | STEME | Stellaria media |
| VERPE | Veronica persica | | |

**Kulturpflanzen**

| | | | |
|---|---|---|---|
| BRSNW | Brassica napus (Raps) | ORYZA | Oryza sativa (Reis) |
| ZEAMX | Zea mays (Mais) | TRZAS | Triticum aestivum (Weizen) |

3a. Tabellen A bis L: Wirkung im Vorauflauf

**Tabelle A: Dosierung 80/ha, Schädigung von Kulturpflanzen**

| Verbindung | ORYZA | TRZAS | BRSNW |
|---|---|---|---|
| 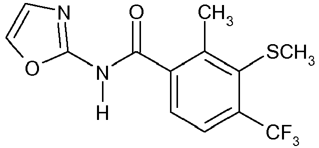 | 0% | 0% | 30% |
| erfindungsgemäß, Nr.1-171 | | | |
| 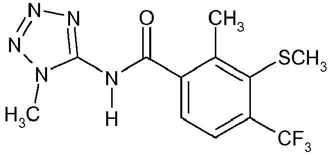 | 60% | 60% | 80% |
| WO 2012/028579, Nr. 4-135 | | | |

**Tabelle B: Dosierung 80/ha, Schädigung von Kulturpflanzen**

| Verbindung | ORYZA | TRZAS | BRSNW |
|---|---|---|---|
| 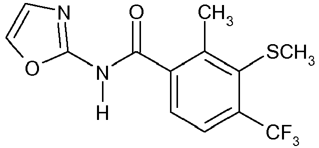 | 0% | 0% | 30% |
| erfindungsgemäß, Nr.1-171 | | | |
| 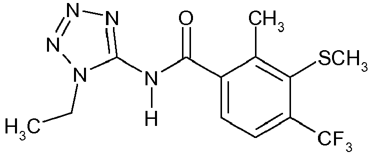 | 90% | 30% | 90% |
| WO 2012/028579, Nr. 5-146 | | | |

**Tabelle C: Dosierung 320/ha, herbizide Wirkung gegen Schadpflanzen**

| Verbindung | ECHCG | ABUTH | AMARE | MATIN |
|---|---|---|---|---|
| 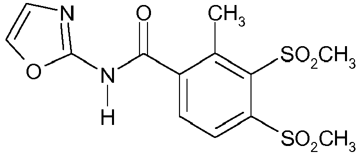 | 70% | 100% | 90% | 100% |
| erfindungsgemäß, Nr.1-163 | | | | |
| 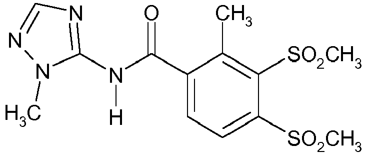 | 0% | 0% | 40% | 40% |
| WO 2012/028579, Nr. 1-188 | | | | |

**Tabelle D: Dosierung 320/ha, herbizide Wirkung gegen Schadpflanzen und Schädigung von Kulturpflanzen**

| Verbindung | Kulturpflanze BRSNW | Schadpflanze ALOMY |
|---|---|---|
| 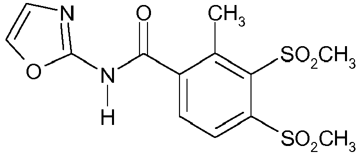 | 10% | 30% |
| erfindungsgemäß, Nr.1-163 | | |
| 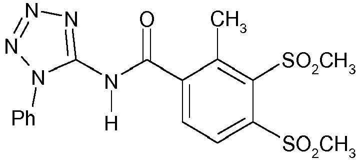 | 90% | 0% |
| WO 2012/028579, Nr. 6-189 | | |

**Tabelle E: Dosierung 320/ha, herbizide Wirkung gegen Schadpflanzen und Schädigung von Kulturpflanzen**

| Verbindung | Kulturpflanzen | | Schadpflanzen | |
|---|---|---|---|---|
| | ORYZA | TRZAS | LOLMU | POLCO |
| 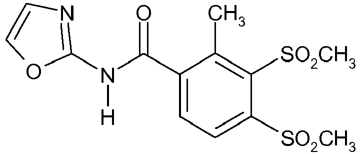 | 0% | 0% | 100% | 70% |
| erfindungsgemäß, Nr.1-163 | | | | |
| 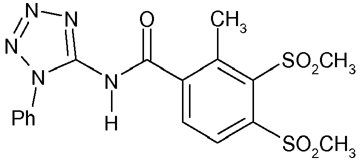 | 80% | 30% | 70% | 0% |
| WO 2012/028579, Nr. 6-189 | | | | |

**Tabelle F: Dosierung 320/ha, herbizide Wirkung gegen Schadpflanzen**

| Verbindung | LOLMU | POLCO |
|---|---|---|
| 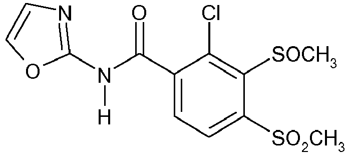 | 100% | 70% |
| erfindungsgemäß, Nr.1-284 | | |
| 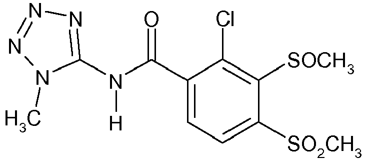 | 20% | 20% |
| WO 2012/028579, Nr. 4-293 | | |

**Tabelle G: Dosierung 320/ha, Schädigung von Kulturpflanzen**

| Verbindung | ORYZA | ZEAMX | BRSNW |
|---|---|---|---|
| 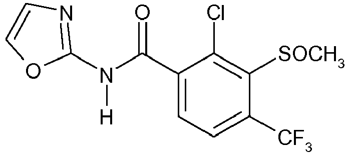 erfindungsgemäß, Nr.1-294 | 30% | 0% | 20% |
| 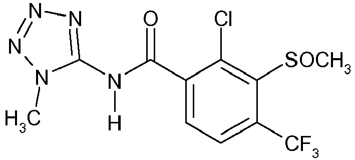 WO 2012/028579 , Nr. 4-639 | 90% | 40% | 90% |

**Tabelle H: Dosierung 80/ha, herbizide Wirkung gegen Schadpflanzen**

| Verbindung | ECGCG | MATIN |
|---|---|---|
| 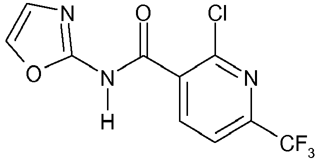 | 70% | 90% |
| erfindungsgemäß, Nr.10-1 | | |
| 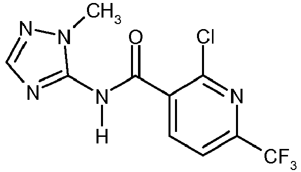 | 0% | 60% |
| WO 2012/028579, Nr. 8-9 | | |

**Tabelle I: Schädigung von ORYZA bei verschiedenen Dosierungen**

| Verbindung | 80 g/ha | 20 g/ha |
|---|---|---|
| 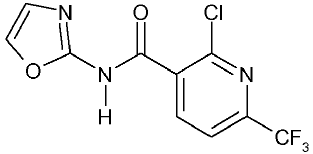 | 0% | 0% |
| erfindungsgemäß, Nr.10-1 | | |
| 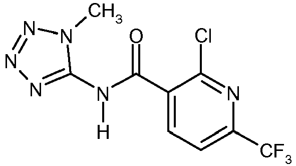 | 90% | 50% |
| WO 2012/028579, Nr. 8-10 | | |

**Tabelle J: herbizide Wirkung gegen AVEFA bei verschiedenen Dosierungen**

| Verbindung | 320 g/ha | 80 g/ha |
|---|---|---|
| 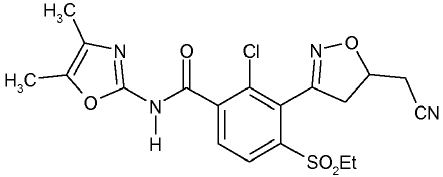 | 100% | 80% |
| erfindungsgemäß, Nr.11-343 | | |
| 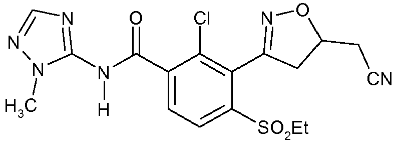 | 0% | 0% |
| WO 2012/028579, Nr. 1-267 | | |

**Tabelle K: herbizide Wirkung gegen AVEFA bei verschiedenen Dosierungen**

| Verbindung | 80 g/ha | 20 g/ha |
|---|---|---|
| 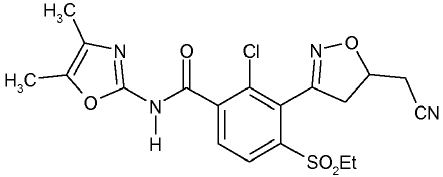 | 80% | 80% |
| erfindungsgemäß, Nr.11-343 | | |
| 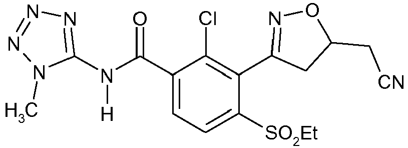 | 0% | 0% |
| WO 2012/028579, Nr. 4-268 | | |

**Tabelle L: herbizide Wirkung gegen AVEFA bei verschiedenen Dosierungen**

| Verbindung | 80 g/ha | 20 g/ha |
|---|---|---|
| 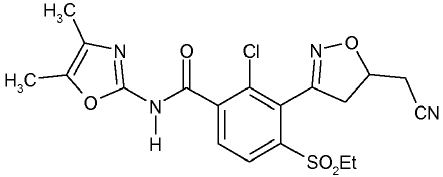 | 80% | 80% |
| erfindungsgemäß, Nr.11-343 | | |
| 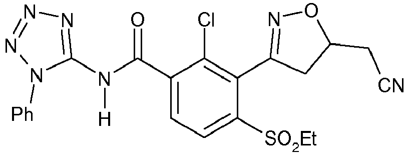 | 0% | 0% |
| WO 2012/028579, Nr. 6-268 | | |

3b. Tabellen M bis X: Wirkung im Nachauflauf

**Tabelle M: Dosierung 80/ha, Schädigung von Kulturpflanzen**

| Verbindung | ORYZA | TRZAS | BRSNW |
|---|---|---|---|
| 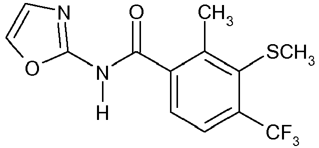 | 0% | 0% | 0% |
| erfindungsgemäß, Nr.1-171 | | | |
| 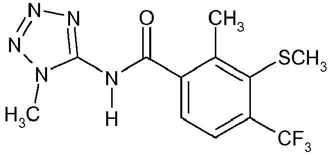 | 100% | 100% | 100% |
| WO 2012/028579, Nr. 4-135 | | | |

**Tabelle N: Dosierung 80/ha, Schädigung von Kulturpflanzen**

| Verbindung | ORYZA | TRZAS | BRSNW |
|---|---|---|---|
| 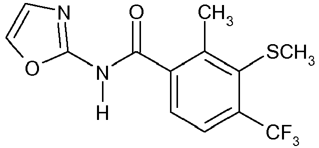 | 0% | 0% | 0% |
| erfindungsgemäß, Nr.1-171 | | | |
| 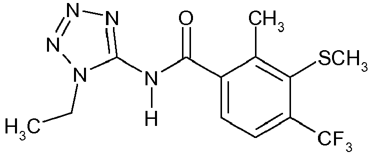 | 80% | 60% | 100% |
| WO 2012/028579, Nr. 5-146 | | | |

**Tabelle O: Dosierung 20/ha, Schädigung von Kulturpflanzen**

| Verbindung | ORYZA | TRZAS | BRSNW |
|---|---|---|---|
| 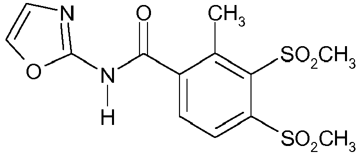 | 0% | 20% | 40% |
| erfindungsgemäß, Nr.1-163 | | | |
| 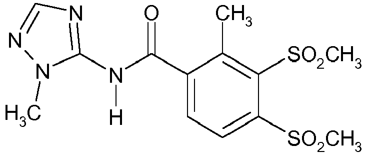 | 20% | 40% | 100% |
| WO 2012/028579, Nr. 1-188 | | | |

**Tabelle P: Dosierung 80/ha, herbizide Wirkung gegen Schadpflanzen**

| Verbindung | ALOMY | AVEFA | LOMU | SETVI |
|---|---|---|---|---|
| 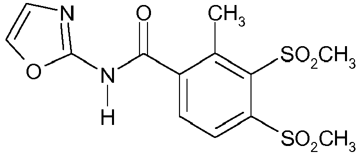 | 60% | 40% | 20% | 100% |
| erfindungsgemäß, Nr.1-163 | | | | |
| 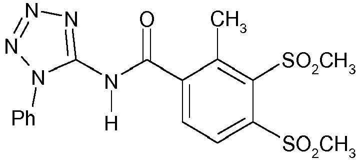 | 0% | 0% | 0% | 20% |
| WO 2012/028579, Nr. 6-189 | | | | |

**Tabelle Q: Dosierung 80/ha, herbizide Wirkung gegen Schadpflanzen und Schädigung von Kulturpflanzen**

| Verbindung | Kulturpflanze ORYZA | Schadpflanzen | |
|---|---|---|---|
| | | CYPES | LOLMU |
| 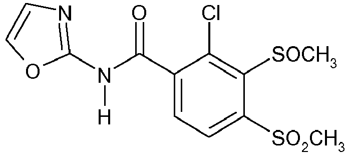 | 20% | 60% | 70% |
| erfindungsgemäß, Nr.1-284 | | | |
| 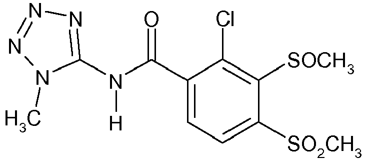 | 40% | 40% | 40% |
| WO 2012/028579, Nr. 4-293 | | | |

**Tabelle R: Dosierung 20/ha, herbizide Wirkung gegen Schadpflanzen**

| Verbindung | LOLMU | SETVI |
|---|---|---|
| 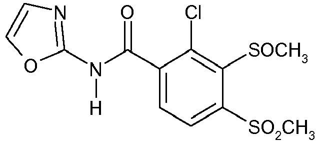 | 60% | 90% |
| erfindungsgemäß, Nr.1-284 | | |
| 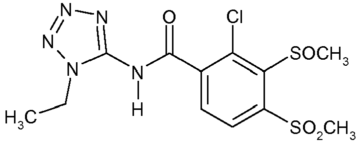 | 10% | 0% |
| WO 2012/028579, Nr. 5-294 | | |

**Tabelle S: Dosierung 20/ha, herbizide Wirkung gegen Schadpflanzen und Schädigung von Kulturpflanzen**

| Verbindung | Kulturpflanzen | | | Schadpflanze |
|---|---|---|---|---|
| | ORYZA | TRZAS | ZEAMX | PHBPU |
| 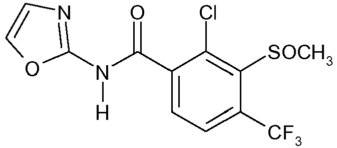 | 0% | 0% | 0% | 70% |
| erfindungsgemäß, Nr.1-294 | | | | |
| 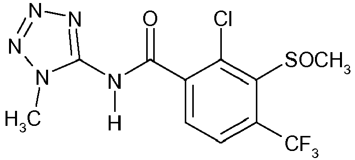 | 60% | 100% | 80% | 40% |
| WO 2012/028579, Nr. 4-639 | | | | |

**Tabelle T: Dosierung 5/ha, herbizide Wirkung gegen Schadpflanzen**

| Verbindung | STEME | VERPE |
|---|---|---|
| 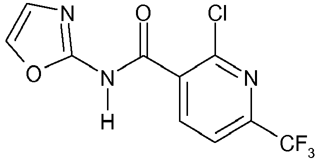 | 40% | 90% |
| erfindungsgemäß, Nr.10-1 | | |
| 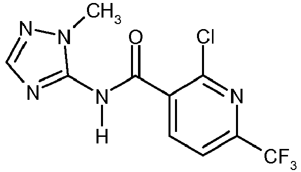 | 10% | 60% |
| WO 2012/028579, Nr. 8-9 | | |

**Tabelle U: Dosierung 80 g/ha, Schädigung von Kulturpflanzen**

| Verbindung | ORYZA | ZEAMX |
|---|---|---|
| 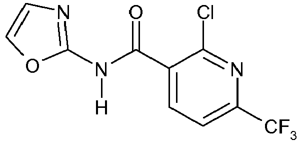 | 0% | 0% |
| erfindungsgemäß, Nr.10-1 | | |
| 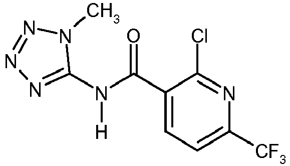 | 90% | 90% |
| WO 2012/028579, Nr. 8-10 | | |

**Tabelle V: Schädigung von BRSNW bei verschiedenen Dosierungen**

| Verbindung | 80 g/ha | 20 g/ha |
|---|---|---|
| 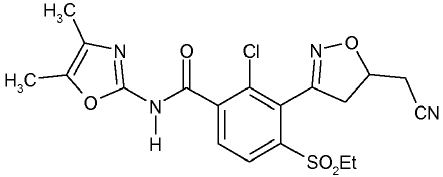 | 0% | 0% |
| erfindungsgemäß, Nr.11-343 | | |
| 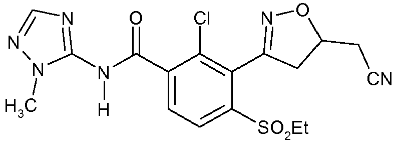 | 90% | 90% |
| WO 2012/028579, Nr. 1-267 | | |

**Tabelle W: Schädigung von BRSNW bei verschiedenen Dosierungen**

| Verbindung | 20 g/ha | 5 g/ha |
|---|---|---|
| 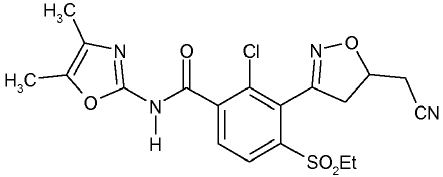 | 0% | 0% |
| erfindungsgemäß, Nr.11-343 | | |
| 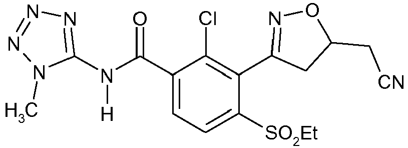 | 100% | 80% |
| WO 2012/028579, Nr. 4-268 | | |

**Tabelle X: Schädigung von BRSNW bei verschiedenen Dosierungen**

| Verbindung | 80 g/ha | 20 g/ha |
|---|---|---|
| 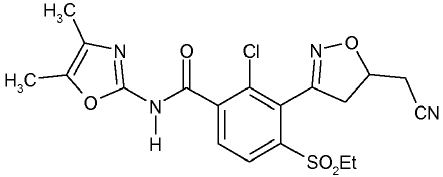 | 0% | 0% |
| erfindungsgemäß, Nr.11-343 | | |
| 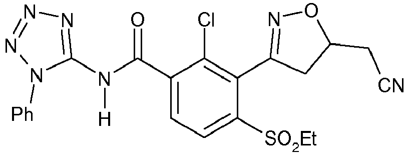 | 60% | 20% |
| WO 2012/028579, Nr. 6-268 | | |

## Patentansprüche

1. N-(Oxazol-2-yl)-arylcarbonsäureamide der Formel (I) oder deren Salze 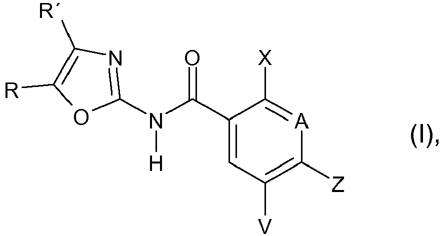 worin
A bedeutet N oder CY,
R und R' bedeuten unabhängig voneinander jeweils Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, (C₂-C₆)-Al kenyl, (C₂-C₆)-Alkenyloxy, (C₂-C₆)-Halogenalkenyl, (C₂-C₆)-Alkinyl, (C₂-C₆)-Alkinyloxy, (C₂-C₆)-Halogenalkinyl, Cyano-(C₁-C₆)-Alkyl, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe Methyl, Ethyl, Methoxy, Trifluormethyl und Halogen substituiertes Heteroaryl, Heterocyclyl oder Phenyl,
X bedeutet Nitro, Halogen, Cyano, Formyl, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(0)NR¹OR¹, OR², OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR₁R₂, P(O)(OR⁵)₂, CH₂P(O)(OR⁵)₂, (C₁-C₆)-Alkyl-Heteroaryl, (C₁-C₆)-Alkyl-Heterocyclyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹,OCOOR¹, NR¹COOR¹, C(O)N(R¹)₂, NR¹C(O)N(R¹)₂, OC(O)N(R¹)₂, CO(NOR¹)R¹, CHNOR¹, CH₂ONCR³)₂, NR¹SO₂R², NR¹COR¹, OR¹, OSO₂R², S(O)ₙR², SO₂OR¹, SO₂N(R¹)₂(C₁-C₆)-Alkyl-S(O)ₙR², NS(O)R⁶R⁷, S(O)R⁸NR⁹, (C₁-C₆)-Alkyl-OR¹, (C₁-C₆)-Alkyl-OCOR¹, (C₁-C₆)-Alkyl-OSO2R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, Nitro, (C₁-C₆)-Alkyl, (C₁-C₆)-Alkoxy, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², NR¹R², P(O)(OR⁵)₂, Heteroaryl, Heterocyclyl oder Phenyl, wobei die letzten drei Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl 0 bis 2 Oxogruppen trägt, oder
Z kann auch Wasserstoff bedeuten, falls Y für den Rest S(O)ₙR² steht,
V bedeutet Wasserstoff, (C₁-C₆)-Alkyl, Halogen-(C₁-C₆)-alkyl, (C₁-C₆)-Alkoxy, (C₁-C₆)-Halogenalkoxy, S(O)ₙ-(C₁-C₆)-Alkyl, S(O)ₙ-(C₁-C₆)-Halogenalkyl, (C₁-C₆)-Alkoxy-(C₁-C₄)-alkyl, Halogen, Nitro oder Cyano,
R¹ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocycl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl wobei die 21 letztgenannten Reste durch s Reste aus der Gruppe bestehend aus Cyano, Halogen, Nitro, Rhodano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R⁴ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Methyl oder Ethyl,
R⁶ und R⁷ bedeuten unabhängig voneinander jeweils (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy-(C₁-C₆)-alkyl, Halogen-(C₁-C₆)-alkoxy-(C₁-C₆)-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
oder
R⁶ und R⁷ bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außerdem Schwefelatom der Sulfoximinogruppe jeweils m Ringglieder aus der Gruppe bestehend aus N(R¹), O und S(O)ₙ enthält, und wobei dieser Ring jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl, substituiert ist, und wobei dieser Ring n Oxogruppen trägt,
R⁸ bedeutet jeweils durch s Reste aus der Gruppe bestehend aus Nitro, Halogen, Cyano, Rhodano, (C₃-C₆)-Cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S und (R⁵O)₂(O)P substituiertes (C₁-C₆)-Alkyl, (C₂-C₆)-Alkenyl oder (C₂-C₆)-Alkinyl,
oder
jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P und R¹O-(C₁-C₆)-Alkyl im cyclischen Teil substituiertes (C₃-C₆)-Cycloalkyl, (C₃-C₆)-Cycloalkenyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R¹)-(C₁-C₆)-alkyl, Heteroaryl- N(R¹)-(C₁-C₆)-alkyl, Heterocyclyl- N(R¹)-(C₁-C₆)-alkyl, Phenyl-S(O)ₙ-(C₁-C₆)-alkyl, Heteroaryl-S(O)n-(C₁-C₆)-alkyl oder Heterocyclyl-S(O)ₙ-(C₁-C₆)-alkyl, und wobei Heterocyclyl n Oxogruppen trägt,
R⁹ bedeutet Wasserstoff, Nitro, Halogen, Cyano, (C₁-C₆)-Alkyl, Halogen-(C₁-C₆)-alkyl, (C₃-C₆)-Alkenyl, Halogen-(C₃-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R²O(O)C, (R¹)₂N(O)C, R²S(O)C, (R¹)₂N(S)C, R¹(R¹O)N(O)C, R²(O)₂S(R¹)N(O)C, (R¹)₂N(O)₂S(R¹)N(O)C, R¹O, (R¹)₂N, R²(O)ₙS, (R²)₃Si-(C₁-C₆)-Alkyl-(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, R²(O)₂S(R¹)N(O)₂S, (R⁵O)₂(O)P, (R²)₃Si, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, (R²)₃Si-(C₁-C₆)-Alkyl,
oder
jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl im cyclischen Teil substituiertes Phenyl, Heteroaryl, Heterocyclyl, Phenyl-(C₁-C₆)-alkyl, Heteroaryl-(C₁-C₆)-alkyl oder Heterocyclyl-(C₁-C₆)-alkyl, und wobei Heterocyclyl n Oxogruppen trägt,
m bedeutet 0, 1, 2, 3 oder 4,
n bedeutet 0, 1 oder 2,
s bedeutet 0, 1, 2 oder 3,
wobei die Verbindungen {[(5-Methoxy-2-{[5-(2,2,2-trifluorethyl)-1,3-oxazol-2-yl]carbamoyl}phenoxy)carbonyl]oxy}methyl-2,2-dimethylpropanoat, Ethyl-4-methyl-2-({[2-methyl-6-(trifluormethyl)pyridin-3-yl]carbonyl}amino)-1,3-oxazol-5-carboxylat, N-(4,5-Dimethyl-1,3-oxazol-2-yl)-2,4-dimethylbenzamid und 2,4-Dichlor-N-(4,5-diphenyl-1,3-oxazol-2-yl)benzamid ausgenommen sind.

2. N-(Oxazol-2-yl)-arylcarbonsäureamide nach Anspruch 1, worin
A bedeutet N oder CY,
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, C₁-C₆)-Alkyl-O-(C₁-C₆)-alkyl, (C₃-C₆)-Cycloalkyl-(C₁-C₆)-alkyl, Halogen-(C₃-C₆)-cycloalkyl-(C₁-C₆)-alkyl, COR¹, OR², OCOR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹ oder (C₁-C₆)-Alkyl-NR¹SO₂R², (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, OR¹, COOR¹, OSO₂R², S(O)ₙR², SO₂OR¹, SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Rhodano, Nitro, (C₁-C₆)-Alkyl, (C₁-C₆)-Alkoxy, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-CyCloalkyl-(C₁-C₆)-alkyl, COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², 1,2,4-Triazol-1-yl, oder
Z kann auch Wasserstoff bedeuten, falls Y für den Rest S(O)ₙR² steht,
V bedeutet Wasserstoff, (C₁-C₆)-Alkyl, Halogen-(C₁-C₆)-alkyl, (C₁-C₆)-Alkoxy, (C₁-C₆)-Halogenalkoxy, S(O)ₙ-(C₁-C₆)-alkyl, S(O)ₙ-(C₁-C₆)-halogenalkyl, (C₁-C₆)-Alkoxy-(C₁-C₄)-alkyl, Halogen, Nitro oder Cyano,
R, R' bedeuten unabhängig voneinander jeweils Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano-(C₁-C₆)-Alkyl, Cyano, Methylsulfenyl, Methylsulfinyl, Methylsulfonyl, Acetylamino, Methoxymethyl, oder
jeweils durch s Reste aus der Gruppe Methyl, Ethyl, Methoxy, Trifluormethyl und Halogen substituiertes Heteroaryl, Heterocyclyl oder Phenyl,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei diese Reste durch s Reste aus der Gruppe bestehend aus Cyano, Halogen, Nitro, OR³, S(O)ₙR⁴, N(R³)₂, NR³OR³, NR³SO₂R⁴, COR³, OCOR³, NR³COR³, CO₂R³, CON(R³)₂ und (C₁-C₄)-Alkoxy-(C₂-C₆)-alkoxycarbonyl substituiert sind, und wobei Heterocyclyl n Oxogruppen trägt,
R³ bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cy_{C}loalkyl-(C₁-C₆)-alkyl,
R⁴ bedeutet (C₁-C₆)-Alkyl, (C₂-C₆)-Alkenyl oder (C₂-C₆)-Alkinyl,
R⁶ und R⁷ bedeuten unabhängig voneinander jeweils (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy-(C₁-C₆)-alkyl, Halogen-(C₁-C₆)-alkoxy-(C₁-C₆)-alkyl, Phenyl, Heteroaryl oder Heterocyclyl, wobei die drei letztgenannten Reste jeweils durch s Reste aus der Gruppe bestehend aus Nitro, Halogen, (C₁-C₆)-Alkyl, Halogen-(C₁-C₆)-alkyl, R¹O(O)C, (R¹)₂N(O)C, R¹O, (R¹)₂N, R²(O)ₙS, und R¹O-(C₁-C₆)-Alkyl substituiert sind, und wobei Heterocyclyl n Oxogruppen trägt,
oder R⁶ und R⁷ bilden gemeinsam mit dem Schwefel-Atom, an dem sie gebunden sind, einen 3- bis 8-gliedrigen ungesättigten, teilgesättigten oder gesättigten Ring, der außer den Kohlenstoffatomen und außer dem Schwefelatom der Sulfoximinogruppe jeweils m Ringglieder aus der Gruppe bestehend aus N(R¹), O und S(O)ₙ enthält, und wobei dieser Ring jeweils durch s Reste aus der Gruppe bestehend aus Halogen, (C₁-C₆)-Alkyl, Halogen-(C₁-C₆)-alkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl, substituiert ist, und wobei dieser Ring n Oxogruppen trägt,
R⁸ bedeutet jeweils durch s Reste aus der Gruppe bestehend aus Halogen, Cyano, (C₃-C₆)-Cycloalkyl, R¹(O)C, R¹(R¹ON=)_{C}, R¹O(O)C, (R¹)₂N(O)C, R²(O)₂S(R¹)N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, R¹(O)C(R¹)N(O)₂S, R²O(O)C(R¹)N(O)₂S und (R¹)₂N(O)C(R¹)N(O)₂S substituiertes (C₁-C₆)-Alkyl oder
jeweils durch s Reste aus der Gruppe bestehend aus Halogen, (C₁-C₆)-Alkyl, Halogen-(C₁-C₆)-alkyl, (C₃-C₆)-Cycloalkyl, R¹O(O)C und (R¹)₂N(O)C substituiertes (C₃-C₆)-Cycloalkyl,
R⁹ bedeutet Wasserstoff, Nitro, Cyano, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-CyCloalkyl-(C₁-C₆)-alkyl, R¹(O)C, R²O(O)C, (R¹)₂N(O)C, R²(O)₂S, R¹(O)C-(C₁-C₆)-Alkyl, R¹O(O)C-(C₁-C₆)-Alkyl, (R¹)₂N(O)C-(C₁-C₆)-Alkyl, R¹O-(C₁-C₆)-Alkyl, (R¹)₂N-(C₁-C₆)-Alkyl oder R²(O)ₙS-(C₁-C₆)-Alkyl,
m bedeutet 0, 1 oder 2,
n bedeutet 0, 1 oder 2,
s bedeutet 0, 1, 2 oder 3.

3. N-(Oxazol-2-yl)-arylcarbonsäureamide nach Anspruch 1 oder 2, worin
A bedeutet N oder CY,
X bedeutet Nitro, Halogen, Cyano, (C₁-C₆)-Alkyl, Halogen-(C₁-C₆)-alkyl, (C₃-C₆)-Cycloalkyl, OR², S(O)ₙR², (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)2, (C₁-C₆)-Alkyl-NR¹COR¹, (C₁-C₆)-Alkyl-NR¹SO₂R², (C₁-C₆)-Alkyl-Heteroaryl, (C₁-C₆)-Alkyl-Heterocyclyl, wobei die beiden letzt genannten Reste jeweils durch s Reste Halogen, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y Wasserstoff, Nitro, Halogen, Cyano, (C₁-C₆)-Alkyl, (C₁-C₆)-Halogenalkyl, OR¹, S(O)ₙR², SO₂N(R¹)₂, N(R¹)₂, NR¹SO₂R², NR¹COR¹, (C₁-C₆)-Alkyl-S(O)ₙR², (C₁-C₆)-Alkyl-OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die letzten 6 Reste jeweils durch s Reste aus der Gruppe Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z bedeutet Halogen, Cyano, Nitro, (C₁-C₆)-Alkyl, (C₁-C₆)-Alkoxy, Halogen-(C₁-C₆)-alkyl, (C₃-C₆)-Cycloalkyl, S(O)ₙR², 1,2,4-Triazol-1-yl, oder Z kann auch Wasserstoff, bedeuten, falls Y für den Rest S(O)ₙR² steht,
V bedeutet Wasserstoff, (C₁-C₆)-Alkyl, Halogen-(C₁-C₆)-alkyl, (C₁-C₆)-Alkoxy, (C₁-C₆)-Halogenalkoxy, S(O)ₙ-(C₁-C₆)-alkyl, S(O)ₙ-(C₁-C₆)-halogenalkyl, (C₁-C₆)-Alkoxy-(C₁-C₄)-alkyl, Halogen, Nitro oder Cyano,
R, R' bedeuten unabhängig von einander jeweils Wasserstoff, (C₁-C₆)-Alkyl, (C₃-C₇)-cyclo-Alkyl, Halogen-(C₁-C₆)-alkyl,(C₁-C₆)-Alkoxy, Halogen-(C₁-C₆)-alkoxy, Cyano-(C₁-C₆)-Alkyl, Cyano, Methylsulfenyl, Methylsulfinyl, Methylsulfonyl, Acetylamino, Halogen, Amino, Methoxymethyl,
R¹ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl, (C₁-C₆)-Alkyl-O-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl oder (C₁-C₆)-Alkyl-NR³-Heterocyclyl, wobei die 16 letztgenannten Reste durch s Reste aus der Gruppe bestehend aus Cyano, Halogen, Nitro, OR³, S(O)ₙR⁴, N(R³)₂, NR³OR³, COR³, OCOR³, NR³COR³, NR³SO₂R⁴, CO₂R³, CON(R³)₂ und (C₁-C₄)-Alkoxy-(C₂-C₆)-alkoxycarbonyl substituiert sind, und wobei Heterocyclyl n Oxogruppen trägt,
R² bedeutet (C₁-C₆)-Alkyl, (C₃-C₆)-Cycloalkyl oder (C₃-C₆)-CyCloalkyl-(C₁-C₆)-alkyl, wobei diese drei vorstehend genannten Reste jeweils durch s Reste aus der Gruppe bestehend aus Halogen und OR³ substituiert sind,
R³ bedeutet Wasserstoff oder (C₁-C₆)-Alkyl,
R⁴ bedeutet (C₁-C₆)-Alkyl,
R⁶ und R⁷ bedeuten unabhängig voneinander jeweils Methyl, Ethyl oder n-Propyl, oder
R⁶ und R⁷ bilden gemeinsam mit dem Schwefel-Atom, an dem sie gebunden sind, einen 5- oder 6-gliedrigen gesättigten Ring, der außer den Kohlenstoffatomen und außer dem Schwefelatom der Sulfoximinogruppe m Sauerstoffatome enthält,
R⁸ bedeutet Methyl, Ethyl oder n-Propyl,
R⁹ bedeutet Wasserstoff oder Cyano,
m bedeutet 0 oder 1,
n bedeutet 0, 1 oder 2,
s bedeutet 0, 1, 2 oder 3.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

7. Herbizide Mittel nach Anspruch 6 enthaltend einen Safener.

8. Herbizide Mittel nach Anspruch 7 enthaltend cyprosulfamid, cloquintocet-mexyl, mefenpyr-diethyl oder isoxadifen-ethyl.

9. Herbizide Mittel nach einem der Ansprüche 6 bis 8 enthaltend ein weiteres Herbizid.

10. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

11. Verwendung von Verbindungen der Formel (I) gemäß einem der Ansprüche 1 bis 3 oder von herbiziden Mitteln nach einem der Ansprüche 4 bis 9 zur Bekämpfung unerwünschter Pflanzen.

12. Verwendung nach Anspruch 11, **dadurch gekennzeichnet, daß** die Verbindungen der Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

## Claims

1. N-(oxazol-2-yl)arylcarboxamide of the formula (I) or a salt thereof 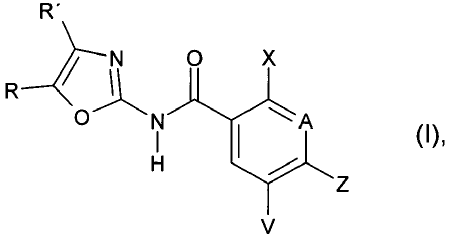 in which
A represents N or CY,
R and R' independently of one another each represent hydrogen, (C₁-C₆)-alkyl, (C₃-C₇)-cycloalkyl, halo-(C₁-C₆-alkyl, (C₁-C₆)-alkoxy, halo-(C₁-C₆)-alkoxy, (C₂-C₆)-alkenyl, (C₂-C₆)-alkenyloxy, (C₂-C₆)-haloalkenyl, (C₂-C₆)-alkynyl, (C₂-C₆)-alkynyloxy, (C₂-C₆)-haloalkynyl, cyano-(C₁-C₆)-alkyl, cyano, nitro, methylsulfenyl, methylsulfinyl, methylsulfonyl, acetylamino, benzoylamino, methoxycarbonyl, ethoxycarbonyl, methoxycarbonylmethyl, ethoxycarbonylmethyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl, or
heteroaryl, heterocyclyl or phenyl, each of which is substituted by s radicals from the group consisting of methyl, ethyl, methoxy, trifluoromethyl and halogen,
X represents nitro, halogen, cyano, formyl, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OR², OCOR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², NR₁R₂, P(O)(OR⁵)₂, CH₂P(O)(OR⁵)₂, (C₁-C₆)-alkyl-heteroaryl, (C₁-C₆)-alkyl-heterocyclyl, where the two last-mentioned radicals are each substituted by s radicals from the group consisting of halogen, (C₁-C₆)-alkyl, halo-(C₁-C₆)-alkyl, S(O)ₙ-(C₁-C₆)-alkyl, (C₁-C₆)-alkoxy and halo-(C₁-C₆)-alkoxy, and where heterocyclyl carries n oxo groups,
Y represents hydrogen, nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, (C₃-C₆)-cycloalkyl, (C₃-C₆)-cycloalkenyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, CHNOR¹, CH₂ONC(R³)₂, NR¹SO₂R², NR¹COR¹, OR¹, OSO₂R², S(O)ₙR², SO₂OR¹, SO₂N(R¹)₂ (C₁-C₆)-alkyl-S(O)ₙR², NS(O)R⁶R⁷, S(O)R⁸NR⁹, (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-CN, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², N(R¹)₂, P(O)(OR⁵)₂, CH₂P(O)(OR⁵)₂, (C₁-C₆)-alkylphenyl, (C₁-C₆)-alkylheteroaryl, (C₁-C₆)-alkylheterocyclyl, phenyl, heteroaryl or heterocyclyl, where the 6 last-mentioned radicals are each substituted by s radicals from the group consisting of halogen, nitro, cyano, (C₁-C₆)-alkyl, halo-(C₁-C₆)-alkyl, (C₃-C₆)-cycloalkyl, S(O)n-(C₁-C₆)-alkyl, (C₁-C₆)-alkoxy, halo-(C₁-C₆)-alkoxy, (C₁-C₆)-alkoxy-(C₁-C₄)-alkyl and cyanomethyl, and where heterocyclyl carries n oxo groups,
Z represents halogen, cyano, thiocyanato, nitro, (C₁-C₆) -alkyl, (C₁-C₆)-alkoxy, halo-(C₁-C₆)-alkyl, (C₂-C₆)-alkenyl, halo-(C₂-C₆)-alkenyl, (C₂-C₆)-alkynyl, halo-(C₂-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², NR¹R², P(O)(OR⁵)₂, heteroaryl, heterocyclyl or phenyl, where the three last-mentioned radicals are each substituted by s radicals from the group consisting of halogen, nitro, cyano, (C₁-C₆)-alkyl, halo- (C₁-C₆)-alkyl, (C₃-C₆)-cycloalkyl, S(O)ₙ-(C₁-C₆)-alkyl, (C₁-C₆)-alkoxy or halo-(C₁-C₆)-alkoxy, and where heterocyclyl carries 0 to 2 oxo groups, or
Z may also represent hydrogen if Y represents the S(O)ₙR² radical,
V represents hydrogen, (C₁-C₆)-alkyl, halo-(C₁-C₆)-alkyl, (C₁-C₆)-alkoxy, (C₁-C₆)-haloalkoxy, S(O)ₙ-(C₁-C₆)-alkyl, S(O)ₙ-(C₁-C₆)-haloalkyl, (C₁-C₆)-alkoxy-(C₁-C₄)-alkyl, halogen, nitro or cyano,
R¹ represents hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆-alkyl, (C₃-C₆)-cycloalkyl- (C₁-C₆)-alkyl, phenyl, phenyl- (C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR³-heteroaryl, (C₁-C₆)-alkyl-NR³-heterocyclyl, where the 21 last-mentioned radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ and (C₁-C₄)-alkoxy-(C₂-C₆)-alkoxycarbonyl, and where heterocyclyl carries n oxo groups,
R² represents (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR³-heteroaryl, (C₁-C₆)-alkyl-NR³-heterocyclyl, where the 21 last-mentioned radicals are substituted by s radicals from the group consisting of cyano, halogen, nitro, thiocyanato, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ and (C₁-C₄) -alkoxy- (C₂-C₆)-alkoxycarbonyl, and where heterocyclyl carries n oxo groups,
R³ represents hydrogen, (C₁-C₆)-alkyl, (C₂-C₆)-alkenyl, (C₂-C₆)-alkynyl, (C₃-C₆)-cycloalkyl or (C₃-C₆)-cycloalkyl-(C₁-C₆)-alkyl,
R⁴ represents (C₁-C₆)-alkyl, (C₂-C₆)-alkenyl, (C₂-C₆)-alkynyl, (C₃-C₆)-cycloalkyl, (C₃-C₆)-cycloalkyl- (C₁-C₆)-alkyl or phenyl,
R⁵ represents methyl or ethyl,
R⁶ and R⁷ independently of one another each represent (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl- (C₁-C₆)-alkyl, halo- (C₃-C₆)-cycloalkyl- (C₁-C₆)-alkyl, (C₁-C₆)-alkoxy-(C₁-C₆)-alkyl, halo-(C₁-C₆)-alkoxy-(C₁-C₆)-alkyl, phenyl, heteroaryl or heterocyclyl, where the three last-mentioned radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl carries n oxo groups,
or
R⁶ and R⁷ together with the sulfur atom to which they are attached form a 3- to 8-membered unsaturated, partly saturated or saturated ring which contains, apart from the carbon atoms and apart from the sulfur atom of the sulfoximino group, in each case m ring members from the group consisting of N(R¹), 0 and S(O)ₙ, and where this ring is in each case substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where this ring carries n oxo groups,
R⁸ represents (C₁-C₆)-alkyl, (C₂-C₆)-alkenyl or (C₂-C₆)-alkynyl, each of which is substituted by s radicals from the group consisting of nitro, halogen, cyano, thiocyanato, (C₃-C₆)-cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S and (R⁵O)₂(O)P,
or
(C₃-C₆)-cycloalkyl, (C₃-C₆)-cycloalkenyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆-alkyl, heteroaryl-O-(C₁-C₆)-alkyl, heterocyclyl-0-(C₁-C₆)-alkyl, phenyl-N(R¹)-(C₁-C₆)-alkyl, heteroaryl-N(R¹)-(C₁-C₆)-alkyl, heterocyclyl-N(R¹)-(C₁-C₆)-alkyl, phenyl-S(O)ₙ-(C₁-C₆)-alkyl, heteroaryl-S(O)ₙ-(C₁-C₆)-alkyl or heterocyclyl-S(O)ₙ-(C₁-C₆)-alkyl, each of which is substituted in the cyclic moiety by s radicals from the group consisting of nitro, halogen, cyano, thiocyanato, (C₁-C₆)-alkyl, halo-(C₁-C₆)-alkyl, (C₃-C₆)-cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P and R¹O-(C₁-C₆)-alkyl, and where heterocyclyl carries n oxo groups,
R⁹ represents hydrogen, nitro, halogen, cyano, (C₁-C₆)-alkyl, halo-(C₁-C₆)-alkyl, (C₃-C₆)-alkenyl, halo-(C₃-C6-alkenyl, (C₂-C₆)-alkynyl, halo-(C₃-C₆)-alkynyl, (C₃-C₆-cycloalkyl, halo-(C₃-C₆)-cycloalkyl, (C₃-C₆)-cycloalkyl-(C₁-C₆)-alkyl, halo-3(C₃-C₆)-cycloalkyl-(C₁-C₆)-alkyl, R¹(O)C, R²O(O)C, (R¹)₂N(O)C, R²S(O)C, (R¹)₂N(S)C, R¹(R¹O)N(O)C, R²(O)₂S(R¹)N(O)C, (R¹)₂N(O)₂S(R¹)N(O)C, R¹O, (R¹)₂N, R²(O)ₙS, (R²)₃Si-(C₁-C₆)-alkyl-(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, R²(O)₂S(R¹)N(O)₂S, (R⁵O)₂(O)P, (R²)₃Si, R¹(O)C-(C₁-C₆)-alkyl, R¹O(O)C-(C₁-C₆)-alkyl, (R¹)₂N(O)C-(C₁-C₆)-alkyl, (R¹O)(R¹)N(O)C-(C₁-C₆)-alkyl, R²(O)₂S(R¹)N(O)C-(C₁-C₆)-alkyl, R¹O(O)₂S(R¹)N(O)C-(C₁-C₆)-alkyl, (R¹)₂N(O)₂S(R¹)N(O)C-(C₁-C₆)-alkyl, R¹O-(C₁-C₆)-alkyl, R¹(O)CO-(C₁-C₆)-alkyl, R²(O)₂SO-(C₁-C₆)-alkyl, R²O(O)CO-(C₁-C₆)-alkyl, (R¹)₂N(O)CO-(C₁-C₆)-alkyl, (R¹)₂N-(C₁-C₆)-alkyl, R¹(O)C(R¹)N-(C₁-C₆)-alkyl, R²(O)₂S(R¹)N-(C₁-C₆)-alkyl, R²O(O)C(R¹)N-(C₁-C₆)-alkyl, (R¹)₂N(O)C(R¹)N-(C₁-C₆)-alkyl, R¹O(O)₂S(R¹)N-(C₁-C₆)-alkyl, (R¹)₂N(O)₂S(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, R¹(O)C(R¹)N(O)₂S-(C₁-C₆)-alkyl, R²O(O)C(R¹)N(O)₂S-(C₁-C₆)-alkyl, (R¹)₂N(O)C(R¹)N(O)₂S-(C₁-C₆)-alkyl, (R⁵O)₂(O)P-(C₁-C₆) -alkyl, (R²)₃Si-(C₁-C₆)-alkyl,
or
phenyl, heteroaryl, heterocyclyl, phenyl-(C₁-C₆)-alkyl, heteroaryl-(C₁-C₆)-alkyl or heterocyclyl-(C₁-C₆)-alkyl, each of which is substituted in the cyclic moiety by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl carries n oxo groups,
m represents 0, 1, 2, 3 or 4,
n represents 0, 1 or 2,
s represents 0, 1, 2 or 3,
except for the compounds {[(5-methoxy-2-{[5-(2,2,2-trifluoroethyl)-1,3-oxazol-2-yl]carbamoyl}phenoxy)carbonyl]oxy}methyl 2,2-dimethylpropanoate, ethyl 4-methyl-2-({[2-methyl-6-(trifluoromethyl)pyridin-3-yl]carbonyl}amino)-1,3-oxazol-5-carboxylate,
N-(4,5-dimethyl-1,3-oxazol-2-yl)-2,4-dimethylbenzamide and 2,4-dichloro-N-(4,5-diphenyl-1,3-oxazol-2-yl)benzamide.

2. N-(oxazol-2-yl)arylcarboxamide according to claim 1 in which
A represents N or CY,
X represents nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₁-C₆)-alkyl-O-(C₁-C₆)-alkyl, (C₃-C₆)-cycloalkyl-(C₁-C₆)-alkyl, halo-(C₃-C₆)-cycloalkyl-(C₁-C₆)-alkyl, COR¹, OR², OCOR¹, OSO₂R², S(O)ₙR², SO₂OR¹, SO₂N(R¹)₂, NR¹SO₂R², NR¹COR¹, (C₁-C₆) - alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹ or (C₁-C₆)-alkyl-NR¹SO₂R², (C₁-C₆)-alkyl-heteroaryl, (C₁-C₆)-alkyl-heterocyclyl, where the two last-mentioned radicals are each by s radicals from the group consisting of halogen, (C₁-C₆)-alkyl, halo-(C₁-C₆)-alkyl, S(O)ₙ-(C₁-C₆)-alkyl, (C₁-C₆)-alkoxy and halo-(C₁-C₆)-alkoxy, and where heterocyclyl carries n oxo groups,
Y represents hydrogen, nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, (C₃-C₆)-cycloalkenyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, OR¹, COOR¹, OSO₂R², S(O)ₙR², SO₂OR¹, SO₂ N(R¹)₂, N(R¹)₂, NR¹SO₂R², NR¹COR¹, (C₁-C₆)-alkyl-S(O)ₙR², (C₁-C₆-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆) -alkyl-NR¹COR¹, (C₁-C₆)-alkyl-NR¹SO₂R², (C₁-C₆) -alkylphenyl, (C₁-C₆)-alkyl-heteroaryl, (C₁-C₆)-alkyl-heterocyclyl, phenyl, heteroaryl or heterocyclyl, where the 6 last-mentioned radicals are each substituted by s radicals from the group consisting of halogen, nitro, cyano, (C₁-C₆)-alkyl, halo-(C₁-C₆)-alkyl, (C₃-C₆) -cycloalkyl, S(O)ₙ-(C₁-C₆) -alkyl, (C₁-C₆)-alkoxy, halo- (C₁-C₆) -alkoxy, (C₁-C₆)-alkoxy-(C₁-C₄)-alkyl and cyanomethyl, and where heterocyclyl carries n oxo groups,
Z represents halogen, cyano, thiocyanato, nitro, (C₁-C₆)-alkyl, (C₁-C₆)-alkoxy, halo- (C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-SO₂OR¹, (C₁-C₆)-alkyl-CON(R¹)₂, (C₁-C₆)-alkyl-SO₂N(R¹)₂, (C₁-C₆) -alkyl-NR¹COR¹, (C₁-C₆) -alkyl-NR¹SO₂R², 1, 2, 4-triazol-1-yl, or
Z may also represent hydrogen if Y represents the S(O)ₙR² radical,
V represents hydrogen, (C₁-C₆) -alkyl, halo-(C₁-C₆)-alkyl, (C₁-C₆)-alkoxy, (C₁-C₆)-haloalkoxy, S(O)ₙ-(C₁-C₆)-alkyl, S(O)ₙ-(C₁-C₆)-haloalkyl, (C₁-C₆)-alkoxy-(C₁-C₄)-alkyl, halogen, nitro or cyano,
R, R' independently of one another each represent hydrogen, (C₁-C₆)-alkyl, (C₃-C₇)-cycloalkyl, halo-(C₁-C₆-alkyl, (C₁-C₆)-alkoxy, halo-(C₁-C₆)-alkoxy, cyano-(C₁-C₆)-alkyl, cyano, methylsulfenyl, methylsulfinyl, methylsulfonyl, acetylamino, methoxymethyl, or heteroaryl, heterocyclyl or phenyl, each of which is substituted by s radicals from the group consisting of methyl, ethyl, methoxy, trifluoromethyl and halogen,
R¹ represents hydrogen, (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl, (C₁-C₆)-alkyl-O-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-0-heterocyclyl, (C₁-C₆)-alkyl-NR³-heteroaryl or (C₁-C₆)-alkyl-NR³-heterocyclyl, where the 16 last-mentioned radicals are substituted by s radicals from the group consisting of cyano, halogen, nitro, OR³, S(O)ₙR⁴, N(R³)₂, NR³OR³, COR³, OCOR³, NR³COR³, NR³SO₂R⁴, CO₂R³, CON(R³)₂ and (C₁-C₄)-alkoxy-(C₂-C₆)-alkoxycarbonyl, and where heterocyclyl carries n oxo groups,
R² represents (C₁-C₆)-alkyl, (C₂-C₆) -alkenyl, (C₂-C₆)-alkynyl, (C₃-C₆)-cycloalkyl, (C₃-C₆)-cycloalkyl-(C₁-C₆)-alkyl, (C₁-C₆)-alkyl-O-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR³-heteroaryl or (C₁-C₆)-alkyl-NR³-heterocyclyl, where these radicals are substituted by s radicals from the group consisting of cyano, halogen, nitro, OR³, S(O)ₙR⁴, N(R³)₂, NR³OR³, NR³SO₂R⁴, COR³, OCOR³, NR³COR³, CO₂R³, CON(R³)₂ and (C₁-C₄)-alkoxy-(C₂-C₆)-alkoxycarbonyl, and where heterocyclyl carries n oxo groups,
R³ represents hydrogen, (C₁-C₆)-alkyl, (C₂-C₆)-alkenyl, (C₂-C₆) -alkynyl, (C₃-C₆) -cycloalkyl or (C₃-C₆)-cycloalkyl-(C₁-C₆)-alkyl,
R⁴ represents (C₁-C₆)-alkyl, (C₂-C₆) -alkenyl or (C₂-C₆)-alkynyl,
R⁶ and R⁷ independently of one another each represent (C₁-C₆)-alkyl, halo-(C₁-C₆)-alkyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, (C₁-C₆)-alkoxy-(C₁-C₆)-alkyl, halo-(C₁-C₆)-alkoxy-(C₁-C₆)-alkyl, phenyl, heteroaryl or heterocyclyl, where the three last-mentioned radicals are each substituted by s radicals from the group consisting of nitro, halogen, (C₁-C₆)-alkyl, halo-(C₁-C₆)-alkyl, R¹O(O)C, (R¹)₂N(O)C, R¹O, (R¹)₂N, R²(O)ₙS and R¹O-(C₁-C₆)-alkyl, and where heterocyclyl carries n oxo groups,
or R⁶ and R⁷ together with the sulfur atom to which they are attached form a 3- to 8-membered unsaturated,
partly saturated or saturated ring which contains, in addition to the carbon atoms and in addition to the sulfur atom of the sulfoximino group, in each case m ring members from the group consisting of N(R¹), 0 and S(O)ₙ, and where this ring is in each case substituted by s radicals from the group consisting of halogen, (C₁-C₆)-alkyl, halo-(C₁-C₆)-alkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where this ring carries n oxo groups,
R⁸ represents (C₁-C₆)-alkyl which is in each case substituted by s radicals from the group consisting of halogen, cyano, (C₃-C₆)-cycloalkyl, R¹(O)C, R¹(R¹ON=)C, R¹O(O)C, (R¹)₂N(O)C, R²(O)₂S(R¹)N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, R¹(O)C(R¹)N(O)₂S, R²O(O)C(R¹)N(O)₂S and (R¹)₂N(O)C(R¹)N(O)₂S or (C₃-C₆)-cycloalkyl which is in each case substituted by s radicals from the group consisting of halogen, (C₁-C₆)-alkyl, halo-(C₁-C₆)-alkyl, (C₃-C₆-cycloalkyl, R¹O(O)C and (R¹)₂N(O)C,
R⁹ represents hydrogen, nitro, cyano, (C₁-C₆)-alkyl, halo-(C₁-C₆)-alkyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, R¹(O)C, R²O(O)C, (R¹)₂N(O)C, R²(O)₂S, R¹(O)C-(C₁-C₆)-alkyl, R¹O(O)C-(C₁-C₆)-alkyl, (R¹)₂N(O)C-(C₁-C₆)-alkyl, R¹O-(C₁-C₆)-alkyl, (R¹)₂N-(C₁-C₆)-alkyl or R²(O)ₙS-(C₁-C₆)-alkyl,
m represents 0, 1 or 2,
n represents 0, 1 or 2,
s represents 0, 1, 2 or 3.

3. N-(oxazol-2-yl)arylcarboxamide according to Claim 1 or 2 in which
A represents N or CY,
X represents nitro, halogen, cyano, (C₁-C₆)-alkyl, halo-(C₁-C₆)-alkyl, (C₃-C₆) -cycloalkyl, OR², S(O)ₙR², (C₁-C₆)-alkyl-S(O)ₙR², (C₁-C₆)-alkyl-OR¹, (C₁-C₆)-alkyl-CON(R¹)₂, (C₁-C₆)-alkyl-SO₂N(R¹)₂, (C₁-C₆) -alkyl-NR¹COR¹, (C₁-C₆)-alkyl-NR¹SO₂R², (C₁-C₆) -alkylheteroaryl, (C₁-C₆)-alkylheterocyclyl, where the two last-mentioned radicals are each substituted by s radicals from the group consisting of halogen, (C₁-C₆)-alkyl, halo-(C₁-C₆)-alkyl, S(O)ₙ-(C₁-C₆)-alkyl, (C₁-C₆)-alkoxy, halo-(C₁-C₆)-alkoxy, and where heterocyclyl carries n oxo groups,
Y hydrogen, nitro, halogen, cyano, (C₁-C₆)-alkyl, (C₁-C₆) -haloalkyl, OR¹, S(O)ₙR², SO₂N(R¹)₂, N(R¹)₂, NR¹SO₂R², NR¹COR¹, (C₁-C₆)-alkyl-S(O)ₙR², (C₁-C₆)-alkyl-OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², (C₁-C₆)-alkylphenyl, (C₁-C₆)-alkylheteroaryl, (C₁-C₆)-alkylheterocyclyl, phenyl, heteroaryl or heterocyclyl, where the 6 last-mentioned radicals are each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo- (C₁-C₆) -alkoxy, (C₁-C₆) -alkoxy- (C₁-C₄) -alkyl and cyanomethyl, and where heterocyclyl carries n oxo groups,
Z represents halogen, cyano, nitro, (C₁-C₆)-alkyl, (C₁-C₆)-alkoxy, halo- (C₁-C₆) -alkyl, (C₃-C₆) -cycloalkyl, S(O)ₙR², 1,2,4-triazol-1-yl, or Z may also represent hydrogen if Y represents the S(O)ₙR² radical,
V represents hydrogen, (C₁-C₆)-alkyl, halo-(C₁-C₆)-alkyl, (C₁-C₆)-alkoxy, (C₁-C₆)-haloalkoxy, S(O)ₙ-(C₁-C₆)-alkyl, S(O)ₙ-(C₁-C₆)-haloalkyl, (C₁-C₆)-alkoxy-(C₁-C₄)-alkyl, halogen, nitro or cyano,
R, R' independently of one another each represent hydrogen, (C₁-C₆)-alkyl, (C₃-C₇)-cycloalkyl, halo-(C₁-C₆)-alkyl, (C₁-C₆)-alkoxy, halo-(C₁-C₆)-alkoxy, cyano-(C₁-C₆)-alkyl, cyano, methylsulfenyl, methylsulfinyl, methylsulfonyl, acetylamino, halogen, amino, methoxymethyl,
R¹ represents hydrogen, (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl, (C₁-C₆)-alkyl-O-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-0-heterocyclyl, (C₁-C₆)-alkyl-NR³-heteroaryl or (C₁-C₆)-alkyl-NR³-heterocyclyl, where the 16 last-mentioned radicals are substituted by s radicals from the group consisting of cyano, halogen, nitro, OR³, S(O)ₙR⁴, N(R³)₂, NR³OR³, COR³, OCOR³, NR³COR³, NR³SO₂R⁴, CO₂R³, CON(R³)₂ and (C₁-C₄)-alkoxy-(C₂-C₆)-alkoxycarbonyl, and where heterocyclyl carries n oxo groups,
R² represents (C₁-C₆)-alkyl, (C₃-C₆)-cycloalkyl or (C₃-C₆)-cycloalkyl-(C₁-C₆)-alkyl, where these three radicals mentioned above are each substituted by s radicals from the group consisting of halogen and OR³,
R³ represents hydrogen or (C₁-C₆)-alkyl,
R⁴ represents (C₁-C₆)-alkyl,
R⁶ and R⁷ independently of one another each represent methyl, ethyl or n-propyl,
or
R⁶ and R⁷ together with the sulfur atom to which they are attached form a 5- or 6-membered saturated ring which, in addition to the carbon atoms and in addition to the sulfur atom of the sulfoximino group, contains m oxygen atoms,
R⁸ represents methyl, ethyl or n-propyl,
R⁹ represents hydrogen or cyano,
m represents 0 or 1,
n represents 0, 1 or 2,
s represents 0, 1, 2 or 3.

4. Herbicidal composition **characterized by** a herbicidally active content of at least one compound of the formula (I) according to any of Claims 1 to 3.

5. Herbicidal composition according to Claim 4 in a mixture with formulation auxiliaries.

6. Herbicidal composition according to Claim 4 or 5, comprising at least one further pesticidally active substance from the group consisting of insecticides, acaricides, herbicides, fungicides, safeners, and growth regulators.

7. Herbicidal composition according to Claim 6, comprising a safener.

8. Herbicidal composition according to Claim 7, comprising cyprosulfamide, cloquintocet-mexyl, mefenpyr-diethyl or isoxadifen-ethyl.

9. Herbicidal composition according to any of Claims 6 to 8, comprising a further herbicide.

10. Method for controlling unwanted plants, **characterized in that** an effective amount of at least one compound of the formula (I) according to any of Claims 1 to 3 or of a herbicidal composition according to any of Claims 4 to 9 is applied to the plants or to the site of the unwanted vegetation.

11. Use of compounds of the formula (I) according to any of Claims 1 to 3 or of herbicidal compositions according to any of Claims 4 to 9 for controlling unwanted plants.

12. Use according to Claim 11, **characterized in that** the compounds of the formula (I) are used for controlling unwanted plants in crops of useful plants.

13. Use according to Claim 12, **characterized in that** the useful plants are transgenic useful plants.

## Revendications

1. N-(oxazol-2-yl)-arylcarboxamides de formule (I) ou leurs sels 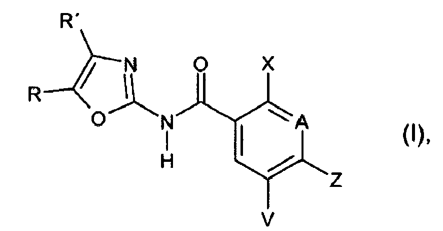 formule dans laquelle
A représente N ou CY,
R et R' représentent chacun indépendamment l'un de l'autre un atome d'hydrogène, un groupe alkyle en C₁-C₆, cycloalkyle en C₃-C₇, halogéno-alkyle(C₁-C₆), alcoxy en C₁-C₆, halogénoalcoxy(C₁-C₆), alcényle en C₂-C₆, alcényloxy en C₂-C₆, halogénoalcényle(C₂-C₆), alcynyle en C₂-C₆, alcynyloxy en C₂-C₆, halogénoalcynyle(C₂-C₆), cyano-alkyle(C₁-C₆), cyano, nitro, méthylsulfényle, méthylsulfinyle, méthylsulfonyle, acétylamino, benzoylamino, méthoxycarbonyle, éthoxycarbonyle, méthoxycarbonylméthyle, éthoxycarbonylméthyle, benzoyle, méthylcarbonyle, pipéridinylcarbonyle, trifluorométhylcarbonyle, un atome d'halogène, un groupe amino, aminocarbonyle, méthylaminocarbonyle, diméthylaminocarbonyle, méthoxyméthyle, ou un groupe hétéroaryle, hétérocyclyle ou phényle chacun substitué par s radicaux choisis dans le groupe constitué par méthyle, éthyle, méthoxy, trifluorométhyle et halogéno,
X représente un groupe nitro, un atome d'halogène, un groupe cyano, formyle, sulfocyano, alkyle en C₁-C₆, halogéno-alkyle(C₁-C₆), alcényle en C₂-C₆, halogéno-alcényle(C₂-C₆), alcynyle en C₂-C₆, halogéno-alcynyle(C₃-C₆), cycloalkyle en C₃-C₆, halogéno-cycloalkyle(C₃-C₆), cycloalkyl (C₃-C₆)-alkyle(C₁-C₆), halogéno-cycloalkyl(C₃-C₆)-alkyle (C₁-C₆), COR¹, COOR¹, OCOOR¹, NR¹COOR¹, C(O)N(R¹)₂, NR¹C(O)N(R¹)₂, OC(O)N(R¹)₂, C(O)NR¹OR¹, OR², OCOR¹, OSO₂R², S(O)ₙR², SO₂OR¹, SO₂N(R¹)₂, NR¹SO₂R², NR¹COR¹, alkyl(C₁-C₆)-S(O)nR², alkyl(C₁-C₆)-OR¹, alkyl(C₁-C₆)-OCOR¹, alkyl(C₁-C₆)-OSO₂R², alkyl(C₁-C₆)-CO₂R¹, alkyl(C₁-C₆)-SO₂OR¹, alkyl(C₁-C₆)-CON(R¹)₂, alkyl(C₁-C₆)-SO₂N(R¹)₂, alkyl(C₁-C₆)-NR¹COR¹, alkyl(C₁-C₆)-NR¹SO₂R², NR₁R₂, P(O)(OR⁵)₂, CH₂P(O)(OR⁵)₂, alkyl(C₁-C₆)-hétéroaryle, alkyl(C₁-C₆)-hétérocyclyle, les deux radicaux nommés en dernier étant substitués chacun par s radicaux choisis dans le groupe constitué par halogéno, alkyle en C₁-C₆, halogéno-alkyle(C₁-C₆), S(O)ₙ-alkyle(C₁-C₆), alcoxy en C₁-C₆ et halogéno-alcoxy(C₁-C₆), et le groupe hétérocyclyle portant n groupes oxo,
Y représente un atome d'hydrogène ou d'halogène, un groupe nitro, cyano, sulfocyano, alkyle en C₁-C₆, halogéno-alkyle(C₁-C₆), alcényle en C₂-C₆, halogéno-alcényle(C₂-C₆), alcynyle en C₂-C₆, halogéno-alcynyle(C₂-C₆), cycloalkyle en C₃-C₆, cycloalcényle en C₃-C₆, halogéno-cycloalkyle(C₃-C₆), cycloalkyl(C₃-C₆)-alkyle(C₁-C₆), halogéno-cycloalkyl (C₃-C₆)-alkyle(C₁-C₆), COR¹, COOR¹, OCOOR¹, NR¹COOR¹, C(O)N(R¹)₂, NR¹C(O)N(R¹)₂, OC(O)N(R¹)₂, CO(NOR¹)R¹, CHNOR¹, CH₂ONCR³)₂, NR¹SO₂R², NR¹COR¹, OR¹, OSO₂R², S(O)ₙR², SO₂OR¹, SO₂N(R¹)₂, alkyl(C₁-C₆)-S(O)ₙR², NS(O)R⁶R⁷, S(O)R⁸NR⁹, alkyl(C₁-C₆)-OR¹, alkyl (C₁-C₆)-OCOR¹, alkyl(C₁-C₆)-OSO₂R², alkyl(C₁-C₆)-CO₂R¹, alkyl(C₁-C₆)-CN, alkyl(C₁-C₆)-SO₂OR¹, alkyl(C₁-C₆)-CON(R¹)₂, alkyl(C₁-C₆)-SO₂N(R¹)₂,
alkyl(C₁-C₆)-NR¹COR¹, alkyl(C₁-C₆)-NR¹SO₂R², N(R¹)₂, P(O)(OR⁵)₂, CH₂P(O)(OR⁵)₂, alkyl(C₁-C₆)-phényle, alkyl(C₁-C₆)-hétéroaryle, alkyl(C₁-C₆)-hétérocyclyle, phényle, hétéroaryle ou hétérocyclyle, les 6 derniers radicaux étant substitués chacun par s radicaux choisis dans le groupe constitué par halogéno, nitro, cyano, alkyle en C₁-C₆, halogéno-alkyle(C₁-C₆), cycloalkyle en C₃-C₆, S(O)ₙ-alkyle(C₁-C₆), alcoxy en C₁-C₆, halogéno-alcoxy(C₁-C₆), alcoxy(C₁-C₆)-alkyle(C₁-C₄) et cyanométhyle, et le groupe hétérocyclyle portant n groupes oxo,
Z représente un atome d'halogène, un groupe cyano, sulfocyano, nitro, alkyle en C₁-C₆, alcoxy en C₁-C₆, halogéno-alkyle(C₁-C₆), alcényle en C₂-C₆, halogéno-alcényle(C₂-C₆), alcynyle en C₂-C₆, halogéno-alcynyle(C₂-C₆), cycloalkyle en C₃-C₆, halogéno-cycloalkyle(C₃-C₆), cycloalkyl(C₃-C₆)-alkyle(C₁-C₆), halogéno-cycloalkyl(C₃-C₆)-alkyle(C₁-C₆), COR¹, COOR¹, OCOOR¹, NR¹COOR¹, C(O)N(R¹)₂, NR¹C(O)N(R¹)₂, OC(O)N(R¹)₂, C(O)NR¹OR¹, OSO₂R², S(O)ₙR², SO₂OR¹, SO₂N(R¹)₂, NR¹SO₂R², NR¹COR¹, alkyl(C₁-C₆)-S(O)ₙR², alkyl(C₁-C₆)-OR¹, alkyl(C₁-C₆)-OCOR¹, alkyl(C₁-C₆)-OSO₂R², alkyl(C₁-C₆)-CO₂R¹, alkyl(C₁-C₆)-SO₂OR¹, alkyl(C₁-C₆)-CON(R¹)₂, alkyl(C₁-C₆)-SO₂N(R¹)₂, alkyl(C₁-C₆)-NR¹COR¹, alkyl(C₁-C₆)-NR¹SO₂R², NR¹R², P(O)(OR⁵)₂, hétéroaryle, hétérocyclyle ou phényle, les trois derniers radicaux étant substitués chacun par s radicaux choisis dans le groupe constitué par halogéno, nitro, cyano, alkyle en C₁-C₆, halogéno-alkyle(C₁-C₆), cycloalkyle en C₃-C₆, S(O)ₙ-alkyle(C₁-C₆), alcoxy en C₁-C₆ ou halogéno-alcoxy(C₁-C₆), et le groupe hétérocyclyle portant 0 ou 2 groupes oxo, ou
Z peut également représenter un atome d'hydrogène, lorsque Y représente le radical S(O)ₙR²,
V représente un atome d'hydrogène, un groupe alkyle en C₁-C₆, halogéno-alkyle(C₁-C₆), alcoxy en C₁-C₆, halogénoalcoxy(C₁-C₆), S(O)ₙ-alkyle(C₁-C₆), S(O)ₙ-halogéno-alkyle(C₁-C₆), alcoxy(C₁-C₆)-alkyle(C₁-C₄), un atome d'halogène, un groupe nitro ou cyano,
R¹ représente un atome d'hydrogène, un groupe alkyle en C₁-C₆, halogéno-alkyle(C₁-C₆), alcényle en C₂-C₆, halogénoalcényle(C₂-C₆), alcynyle en C₂-C₆, halogéno-alcynyle(C₂-C₆), cycloalkyle en C₃-C₆, cycloalcényle en C₃-C₆, halogéno-cycloalkyle(C₃-C₆), alkyl(C₁-C₆)-O-alkyle(C₁-C₆), cycloalkyl(C₃-C₆)-alkyle(C₁-C₆), phényle, phényl-alkyle(C₁-C₆), hétéroaryle, alkyl(C₁-C₆)-hétéroaryle, hétérocyclyle, alkyl(C₁-C₆)-hétérocyclyle, alkyl(C₁-C₆)-O-hétéroaryle, alkyl(C₁-C₆)-O-hétérocyclyle, alkyl(C₁-C₆)-NR³-hétéroaryle, alkyl(C₁-C₆)-NR³-hétérocyclyle, les 21 radicaux nommés en dernier étant substitués par s radicaux choisis dans le groupe constitué par cyano, halogéno, nitro, sulfocyano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ et alcoxy(C₁-C₄)-alcoxycarbonyle(C₂-C₆) et le groupe hétérocyclyle portant n groupes oxo,
R² représente un groupe alkyle en C₁-C₆, halogéno-alkyle(C₁-C₆), alcényle en C₂-C₆, halogéno-alcényle(C₂-C₆), alcynyle en C₂-C₆, halogéno-alcynyle(C₂-C₆), cycloalkyle en C₃-C₆, cycloalcényle en C₃-C₆, halogéno-cycloalkyle(C₃-C₆), alkyl(C₁-C₆)-O-alkyle(C₁-C₆), cycloalkyl(C₃-C₆)-alkyle(C₁-C₆), phényle, phényl-alkyle(C₁-C₆), hétéroaryle, alkyl(C₁-C₆)-hétéroaryle, hétérocyclyle, alkyl(C₁-C₆)-hétérocyclyle, alkyl(C₁-C₆)-O-hétéroaryle, alkyl(C₁-C₆)-O-hétérocyclyle, alkyl(C₁-C₆)-NR³-hétéroaryle, alkyl(C₁-C₆)-NR³-hétérocyclyle, les 21 radicaux nommés en dernier étant substitués par s radicaux choisis dans le groupe constitué par cyano, halogéno, nitro, sulfocyano, OR³, S(O)ₙR⁴, N(R³)₂, NR³OR³, COR³, OCOR³, SCOR⁴, NR³COR³, NR³SO₂R⁴, CO₂R³, COSR⁴, CON(R³)₂ et alcoxy(C₁-C₄)-alcoxycarbonyle (C₂-C₆) et le groupe hétérocyclyle portant n groupes oxo,
R³ représente un atome d'hydrogène, un groupe alkyle en C₁-C₆, alcényle en C₂-C₆, alcynyle en C₂-C₆, cycloalkyle en C₃-C₆ ou cycloalkyl(C₃-C₆)-alkyle(C₁-C₆),
R⁴ représente un groupe alkyle en C₁-C₆, alcényle en C₂-C₆, alcynyle en C₂-C₆, cycloalkyle en C₃-C₆, cycloalkyl(C₃-C₆)-alkyle(C₁-C₆) ou phényle,
R⁵ représente le groupe méthyle ou éthyle,
R⁶ et R⁷ représentent chacun indépendamment l'un de l'autre un groupe alkyle en C₁-C₆, halogéno-alkyle(C₁-C₆), alcényle en C₂-C₆, halogéno-alcényle(C₂-C₆), alcynyle en C₂-C₆, halogéno-alcynyle(C₃-C₆), cycloalkyle en C₃-C₆, halogéno-cycloalkyle(C₃-C₆), cycloalkyle(C₃-C₆)-alkyle(C₁-C₆), halogéno-cycloalkyl(C₃-C₆)-alkyle(C₁-C₆), alcoxy(C₁-C₆)-alkyle(C₁-C₆), halogéno-alcoxy(C₁-C₆)-alkyle(C₁-C₆), phényle, hétéroaryle ou hétérocyclyle, les trois radicaux nommés en dernier étant substitués chacun par s radicaux choisis dans le groupe constitué par nitro, halogéno, cyano, sulfocyano, alkyle en C₁-C₆, halogéno-alkyle(C₁-C₆), cycloalkyle en C₃-C₆, R¹O(O)C, (R¹)₂N(O)C, R¹O, (R¹)₂N, R²(O)ₙS, R¹O(O)₂S, (R¹)₂N(O)₂S et R¹O-alkyle(C₁-C₆), et le groupe hétérocyclyle portant n groupes oxo,
ou
R⁶ et R⁷ forment ensemble avec l'atome de soufre, auquel ils sont liés, un cycle à 3 à 8 chaînons, saturé, partiellement saturé ou insaturé qui outre les atomes de carbone et outre l'atome de soufre du groupe sulfoximino contient chaque fois m chaînons formant le cycle, choisis dans le groupe constitué par N(R¹), O et S(O)ₙ, et ce cycle étant chaque fois substitué par s radicaux choisis dans le groupe constitué par nitro, halogéno, cyano, sulfocyano, alkyle en C₁-C₆, halogéno-alkyle(C₁-C₆), cycloalkyle en C₃-C₆, R¹O(O)C, (R¹)₂N(O)C, R¹O, (R¹)₂N, R²(O)ₙS, R¹O(O)₂S, (R¹)₂N(O)₂S et R¹O-alkyle(C₁-C₆), et ce cycle portant n groupes oxo,
R⁸ représente un groupe alkyle en C₁-C₆, alcényle en C₂-C₆ ou alcynyle en C₂-C₆ chacun substitué par s radicaux choisis dans le groupe constitué par nitro, halogéno, cyano, sulfocyano, cycloalkyle en C₃-C₆, R¹(O)C, R¹(R¹ON=)C, R¹O(O)C, (R¹)₂N(O)C, R¹(R¹O)N (0) C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S et (R⁵O)₂(O)P,
ou
représente un groupe cycloalkyle en C₃-C₆, cycloalcényle en C₃-C₆, phényle, phényl-alkyle(C₁-C₆), hétéroaryle, hétéroaryl-alkyle(C₁-C₆), hétérocyclyle, hétéocyclyl-alkyle(C₁-C₆), phényl-O-alkyle(C₁-C₆), hétéroaryl-O-alkyle(C₁-C₆), hétérocyclyl-O-alkyle(C₁-C₆), phényl-N(R¹)-alkyle(C₁-C₆), hétéroaryl-N(R¹)-alkyle(C₁-C₆), hétérocyclyl-N(R¹)-alkyle(C₁-C₆), phényl-S(O)ₙ-alkyle(C₁-C₆), hétéroaryl-S(O)ₙ-alkyle(C₁-C₆) ou hétérocyclyl-S(O)ₙ-alkyle(C₁-C₆), chacun substitué sur le fragment cyclique par s radicaux choisis dans le groupe constitué par nitro, halogéno, cyano, sulfocyano, alkyle en C₁-C₆, halogéno-alkyle(C₁-C₆), cycloalkyle en C₃-C₆, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P et R¹O-alkyle(C₁-C₆), et le groupe hétérocyclyle portant n groupes oxo,
R⁹ représente un atome d'hydrogène ou d'halogène, un groupe nitro, cyano, alkyle en C₁-C₆, halogéno-alkyle(C₁-C₆), alcényle en C₃-C₆, halogéno-alcényle(C₃-C₆), alcynyle en C₂-C₆, halogéno-alcynyle(C₃-C₆), cycloalkyle en C₃-C₆, halogéno-cycloalkyle(C₃-C₆), cycloalkyl(C₃-C₆)-alkyle(C₁-C₆), halogéno-cycloalkyl(C₃-C₆)-alkyle(C₁-C₆), R¹(O)C, R²O(O)C, (R¹)₂N(O)C, R²S(O)C, (R¹)₂N(S)C, R¹(R¹O)N(O)C, R²(O)₂S(R¹)N(O)C, (R¹)₂N(O)₂S(R¹)N(O)C, R¹O, (R¹)₂N, R²(O)ₙS, (R²)₃Si-alkyl(C₁-C₆)-(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, R²(O)₂S(R¹)N(O)₂S, (R⁵O)₂(O)P, (R²)₃Si, R¹(O)C-alkyle(C₁-C₆), R¹O(O)C-alkyle(C₁-C₆), (R¹)₂N(O)C-alkyle(C₁-C₆), (R¹O)(R¹)N(O)C-alkyle(C₁-C₆), R²(O)₂S(R¹)N(O)C-alkyle(C₁-C₆), R¹O(O)₂S(R¹)N(O)C-alkyle(C₁-C₆), (R¹)₂N(O)₂S(R¹)N(O)C-alkyle(C₁-C₆), R¹O-alkyle(C₁-C₆), R¹(O)CO-alkyle(C₁-C₆), R²(O)₂SO-alkyle(C₁-C₆), R²O(O)CO-alkyle(C₁-C₆), (R¹)₂N(O)CO-alkyle(C₁-C₆), (R¹)₂N-alkyle(C₁-C₆), R¹(O)C(R¹)N-alkyle(C₁-C₆), R²(O)₂S(R¹)N-alkyle(C₁-C₆), R²O(O)C(R¹)N-alkyle(C₁-C₆), (R¹)₂N(O)C(R¹)N-alkyle(C₁-C₆), R¹O(O)₂S(R¹)N-alkyle(C₁-C₆), (R¹)₂N(O)₂S(R¹)N-alkyle(C₁-C₆), R²(O)ₙS-alkyle(C₁-C₆), R¹O(O)₂S-alkyle(C₁-C₆), (R¹)₂N(O)₂S-alkyle(C₁-C₆), R¹(O)C(R¹)N(O)₂S-alkyle(C₁-C₆), R²O(O)C(R¹)N(O)₂S-alkyle(C₁-C₆), (R¹)₂N(O)C(R¹)N(O)₂S-alkyle(C₁-C₆), (R⁵O)₂(O)P-alkyle(C₁-C₆), (R²)₃Si-alkyle(C₁-C₆),
ou
un groupe phényle, hétéroaryle, hétérocyclyle, phényl-alkyle(C₁-C₆), hétéroaryl-alkyle(C₁-C₆) ou hétérocyclyl-alkyle(C₁-C₆), chacun substitué sur le fragment cyclique par s radicaux choisis dans le groupe constitué par nitro, halogéno, cyano, sulfocyano, alkyle en C₁-C₆, halogéno-alkyle(C₁-C₆), cycloalkyle en C₃-C₆, R¹O(O)C, (R¹)₂N(O)C, R¹O, (R¹)₂N, R²(O)ₙS, R¹O(O)₂S, (R¹)₂N(O)₂S et R¹O-alkyle(C₁-C₆) et le groupe hétérocyclyle portant n groupes oxo,
m représente 0, 1, 2, 3 ou 4,
n représente 0, 1 ou 2,
s représente 0, 1, 2 ou 3,
les composés propionate de {[(5-méthoxy-2-{[5-(2,2,2-trifluoroéthyl)-1,3-oxazol-2-yl]carbamoyl}phenoxy)-carbonyl]oxy}méthyl-2,2-diméthyle, 4-méthyl-2-({[2-méthyl-6-(trifluorométhyl)pyridin-3-yl]carbonyl}amino)-1,3-oxazole-5-carboxylate d'éthyle, N-(4,5-diméthyl-1,3-oxazol-2-yl)-2,4-diméthylbenzamide et 2,4-dichloro-N-(4,5-diphényl-1,3-oxazol-2-yl)benzamide étant exclus.

2. N-(oxazol-2-yl)-arylcarboxamides selon la revendication 1, dans lesquels
A représente N ou CY,
X représente un groupe nitro, un atome d'halogène, un groupe cyano, sulfocyano, alkyle en C₁-C₆, halogéno-alkyle(C₁-C₆), alcényle en C₂-C₆, halogéno-alcényle(C₂-C₆), alcynyle en C₂-C₆, halogéno-alcynyle(C₃-C₆), cycloalkyle en C₃-C₆, halogéno-cycloalkyle(C₃-C₆), alkyl(C₁-C₆)-O-alkyle(C₁-C₆), cycloalkyl(C₃-C₆)-alkyle(C₁-C₆), halogéno-cycloalkyl(C₃-C₆)-alkyle(C₁-C₆), COR¹, OR², OCOR¹, OSO₂R², S(O)ₙR², SO₂OR¹, SO₂N(R¹)₂, NR¹SO₂R², NR¹COR¹, alkyl(C₁-C₆)-S(O)ₙR², alkyl(C₁-C₆)-OR¹, alkyl(C₁-C₆)-OCOR¹, alkyl(C₁-C₆)-OSO₂R², alkyl (C₁-C₆)-CO₂R¹, alkyl (C₁-C₆)-SO₂OR¹, alkyl(C₁-C₆)-CON(R¹)₂, alkyl(C₁-C₆)-SO₂N(R¹)₂, alkyl(C₁-C₆)-NR¹COR¹ ou alkyl(C₁-C₆)-NR¹SO₂R², alkyl(C₁-C₆)-hétéroaryle, alkyl(C₁-C₆)-hétérocyclyle, les deux radicaux nommés en dernier étant chacun par s radicaux halogéno, alkyle en C₁-C₆, halogéno-alkyle(C₁-C₆), S(O)ₙ-alkyle(C₁-C₆), alcoxy en C₁-C₆, halogéno-alcoxy(C₁-C₆), et le groupe hétérocyclyle portant n groupes oxo,
Y représente un atome d'hydrogène ou d'halogène, un groupe nitro, cyano, sulfocyano, alkyle en C₁-C₆, halogéno-alkyle (C₁-C₆), alcényle en C₂-C₆, halogéno-alcényle(C₂-C₆), alcynyle en C₂-C₆, halogéno-alcynyle(C₃-C₆), cycloalkyle en C₃-C₆, cycloalcényle en C₃-C₆, halogéno-cycloalkyle(C₃-C₆), cycloalkyl (C₃-C₆)-alkyle(C₁-C₆), halogéno-cycloalkyl(C₃-C₆) -alkyle(C₁-C₆), COR¹, OR¹, COOR¹, OSO₂R², S(O)ₙR², SO₂OR¹, SO₂N(R¹)₂, N(R¹)₂, NR¹SO₂R², NR¹COR¹, alkyl (C₁-C₆)-S(O)ₙR², alkyl(C₁-C₆)-OR¹, alkyl(C₁-C₆)-OCOR¹, alkyl(C₁-C₆)-OSO₂R², alkyl(C₁-C₆)-CO₂R¹, alkyl(C₁-C₆)-SO₂OR¹, alkyl(C₁-C₆)-CON(R¹)₂, alkyl(C₁-C₆)-SO₂N(R¹)₂, alkyl (C₁-C₆)-NR¹COR¹, alkyl(C₁-C₆)-NR¹SO₂R², alkyl(C₁-C₆) -phényle, alkyl(C₁-C₆)-hétéroaryle, alkyl(C₁-C₆)-hétérocyclyle, phényle, hétéroaryle ou hétérocyclyle, les 6 derniers radicaux étant substitués chacun par s radicaux choisis dans le groupe constitué par halogéno, nitro, cyano, alkyle en C₁-C₆, halogéno-alkyle(C₁-C₆), cycloalkyle en C₃-C₆, S(O)ₙ-alkyle(C₁-C₆), alcoxy en C₁-C₆, halogénoalcoxy(C₁-C₆), alcoxy(C₁-C₆)-alkyle(C₁-C₄) et cyanométhyle, et le groupe hétérocyclyle portant n groupes oxo,
Z représente un atome d'halogène, un groupe cyano, sulfocyano, nitro, alkyle en C₁-C₆, alcoxy en C₁-C₆, halogéno-alkyle (C₁-C₆), alcényle en C₂-C₆, halogéno-alcényle(C₂-C₆), alcynyle en C₂-C₆, halogéno-alcynyle(C₃-C₆), cycloalkyle en C₃-C₆, halogéno-cycloalkyle(C₃-C₆), cycloalkyl (C₃-C₆)-alkyle(C₁-C₆), halogéno-cycloalkyl(C₃-C₆)-alkyle(C₁-C₆), COR¹, COOR¹, C(O)N(R¹)₂, C(O)NR¹OR¹, OSO₂R², S(O)ₙR², SO₂OR¹, SO₂N(R¹)₂, NR¹SO₂R², NR¹COR¹, alkyl(C₁-C₆)-S(O)ₙR², alkyl(C₁-C₆)-OR¹, alkyl(C₁-C₆) -OCOR¹, alkyl(C₁-C₆) -OS₂R², alkyl(C₁-C₆)-CO₂R¹, alkyl(C₁-C₆)-SO₂OR¹, alkyl(C₁-C₆)-CON(R¹)₂, alkyl(C₁-C₆)-SO₂N(R¹)₂, alkyl(C₁-C₆)-NR¹COR¹, alkyl(C₁-C₆)-NR¹SO₂R², 1,2,4-triazol-1-yle, ou
Z peut également représenter un atome d'hydrogène, lorsque Y représente le radical S(O)ₙR²,
V représente un atome d'hydrogène, un groupe alkyle en C₁-C₆, halogéno-alkyle(C₁-C₆), alcoxy en C₁-C₆, halogénoalcoxy(C₁-C₆), S(O)ₙ-alkyle(C₁-C₆), S(O)ₙ-halogéno-alkyle(C₁-C₆), alcoxy(C₁-C₆)-alkyle(C₁-C₄), un atome d'halogène, un groupe nitro ou cyano,
R, R' représentent chacun indépendamment l'un de l'autre un atome d'hydrogène, un groupe alkyle en C₁-C₆, cycloalkyle en C₃-C₇, halogéno-alkyle(C₁-C₆), alcoxy en C₁-C₆, halogénoalcoxy(C₁-C₆), cyano-alkyle(C₁-C₆), cyano, méthylsulfényle, méthylsulfinyle, méthylsulfonyle, acétylamino, méthoxyméthyle, ou
un groupe hétéroaryle, hétérocyclyle ou phényle chacun substitué par s radicaux choisis dans le groupe constitué par méthyle, éthyle, méthoxy, trifluorométhyle et halogéno,
R¹ représente un atome d'hydrogène, un groupe alkyle en C₁-C₆, alcényle en C₂-C₆, alcynyle en C₂-C₆, cycloalkyle en C₃-C₆, cycloalkyl(C₃-C₆)-alkyle(C₁-C₆), alkyl(C₁-C₆)-O-alkyle(C₁-C₆), phényle, phényl-alkyle(C₁-C₆), hétéroaryle, alkyl (C₁-C₆) -hétéroaryle, hétérocyclyle, alkyl(C₁-C₆)-hétérocyclyle, alkyl(C₁-C₆)-0-hétéroaryle, alkyl(C₁-C₆)-O-hétérocyclyle, alkyl (C₁-C₆)-NR³-hétéroaryle ou alkyl (C₁-C₆)-NR³-hétérocyclyle, les 16 radicaux nommés en dernier étant substitués par s radicaux choisis dans le groupe constitué par cyano, halogéno, nitro, OR³, S(O)ₙR⁴, N(R³)₂, NR³OR³, COR³, OCOR³, NR³COR³, NR³SO₂R⁴, CO₂R³, CON(R³)₂ et alcoxy (C₁-C₄)-alcoxycarbonyle (C₂-C₆) et le groupe hétérocyclyle portant n groupes oxo,
R² représente un groupe alkyle en C₁-C₆, alcényle en C₂-C₆, alcynyle en C₂-C₆, cycloalkyle en C₃-C₆, cycloalkyl(C₃-C₆)-alkyle(C₁-C₆), alkyl(C₁-C₆)-O-alkyle(C₁-C₆), phényle, phényl-alkyle(C₁-C₆), hétéroaryle, alkyl(C₁-C₆)-hétéroaryle, hétérocyclyle, alkyl(C₁-C₆)-hétérocyclyle, alkyl(C₁-C₆)-O-hétéroaryle, alkyl(C₁-C₆)-O-hétérocyclyle, alkyl(C₁-C₆) -NR³-hétéroaryle ou alkyl(C₁-C₆)-NR³-hétérocyclyle, ces radicaux étant substitués par s radicaux choisis dans le groupe constitué par cyano, halogéno, nitro, OR³, S(O)ₙR⁴, N(R³)₂, NR³OR³, NR³SO₂R⁴, COR³, OCOR³, NR³COR³, CO₂R³, CON(R³)₂ et alcoxy(C₁-C₄)-alcoxycarbonyle(C₂-C₆) et le groupe hétérocyclyle portant n groupes oxo,
R³ représente un atome d'hydrogène, un groupe alkyle en C₁-C₆, alcényle en C₂-C₆, alcynyle en C₂-C₆, cycloalkyle en C₃-C₆ ou cycloalkyl(C₃-C₆)-alkyle(C₁-C₆), R⁴ représente un groupe alkyle en C₁-C₆, alcényle en C₂-C₆ ou alcynyle en C₂-C₆,
R⁶ et R⁷ représentent chacun indépendamment l'un de l'autre un groupe alkyle en C₁-C₆, halogéno-alkyle(C₁-C₆), cycloalkyle en C₃-C₆, halogéno-cycloalkyle(C₃-C₆), cycloalkyl(C₃-C₆)-alkyle(C₁-C₆), halogéno-cycloalkyl(C₃-C₆)-alkyle(C₁-C₆), alcoxy(C₁-C₆)-alkyle(C₁-C₆), halogéno-alcoxy(C₁-C₆)-alkyle(C₁-C₆), phényle, hétéroaryle ou hétérocyclyle, les trois radicaux nommés en dernier étant substitués chacun par s radicaux choisis dans le groupe constitué par nitro, halogéno, alkyle en C₁-C₆, halogéno-alkyle(C₁-C₆), R¹O(O)C, (R¹)₂N(O)C, R¹O, (R¹)₂N, R²(O)ₙS et R¹O-alkyle(C₁-C₆), et le groupe hétérocyclyle portant n groupes oxo,
ou
R⁶ et R⁷ forment ensemble avec l'atome de soufre, auquel ils sont liés, un cycle à 3 à 8 chaînons, saturé, partiellement saturé ou insaturé qui outre les atomes de carbone et outre l'atome de soufre du groupe sulfoximino contient chaque fois m chaînons formant le cycle, choisis dans le groupe constitué par N(R¹), O et S(O)ₙ, et ce cycle étant chaque fois substitué par s radicaux choisis dans le groupe constitué par halogéno, alkyle en C₁-C₆, halogéno-alkyle (C₁-C₆), R¹O(O)C, (R¹)₂N(O)C, R¹O, (R¹)2N, R²(O)ₙS, R¹O(O)₂S, (R¹)₂N(O)₂S et R¹O-alkyle(C₁-C₆), et ce cycle portant n groupes oxo,
R⁸ représente un groupe alkyle en C₁-C₆ substitué par s radicaux choisis dans le groupe constitué par halogéno, cyano, cycloalkyle en C₃-C₆, R¹(O)C, R¹(R¹ON=)C, R¹O(O)C, (R¹)₂N(O)C, R²(O)₂S(R¹)N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, R¹(O)C(R¹)N(O)₂S, R²O(O)C(R¹)N(O)₂S et (R¹)₂N(O)C(R¹)N(O)₂S ou
un groupe cycloalkyle en C₃-C₆ substitué par s radicaux choisis dans le groupe constitué par halogéno, alkyle en C₁-C₆, halogéno-alkyle(C₁-C₆), cycloalkyle en C₃-C₆, R¹O(O)C et (R¹)₂N(O)C,
R⁹ représente un atome d'hydrogène, un groupe nitro, cyano, alkyle en C₁-C₆, halogéno-alkyle(C₁-C₆), cycloalkyle en C₃-C₆, halogéno-cycloalkyle(C₃-C₆), cycloalkyl(C₃-C₆)-alkyle(C₁-C₆), halogéno-cycloalkyl(C₃-C₆)-alkyle(C₁-C₆), R¹(O)C, R²O(O)C, (R¹)₂N(O)C, R²(O)₂S, R¹(O)C-alkyle(C₁-C₆), R¹O(O)C-alkyle(C₁-C₆), (R¹)₂N(O)C-alkyle(C₁-C₆), R¹O-alkyle(C₁-C₆), (R¹)₂N-alkyle(C₁-C₆) ou R²(O)ₙS-alkyle(C₁-C₆),
m représente 0, 1 ou 2,
n représente 0, 1 ou 2,
s représente 0, 1, 2 ou 3,

3. N-(oxazol-2-yl)-arylcarboxamides selon la revendication 1 ou 2, dans lesquels
A représente N ou CY,
X représente un groupe nitro, un atome d'halogène, un groupe cyano, alkyle en C₁-C₆, halogéno-alkyle(C₁-C₆), cycloalkyle en C₃-C₆, OR², S(O)ₙR², alkyl(C₁-C₆)-S(O)ₙR², alkyl(C₁-C₆)-OR¹, alkyl(C₁-C₆) -CON(R¹)₂, alkyl(C₁-C₆)-SO₂N(R¹)₂, alkyl(C₁-C₆)-NR¹COR¹, alkyl(C₁-C₆)-N¹SO₂R², alkyl(C₁-C₆)-hétéroaryle, alkyl(C₁-C₆)-hétérocyclyle, les deux radicaux nommés en dernier étant substitués chacun par s radicaux halogéno, alkyle en C₁-C₆, halogéno-alkyle(C₁-C₆), S(O)ₙ-alkyle(C₁-C₆), alcoxy en C₁-C₆, halogéno-alcoxy(C₁-C₆), et le groupe hétérocyclyle portant n groupes oxo,
Y un atome d'hydrogène ou d'halogène, un groupe nitro, cyano, alkyle en C₁-C₆, halogéno-alkyle(C₁-C₆), OR¹, S(O)ₙR², SO₂N(R¹)₂, N(R¹)₂, NR¹SO₂R², NR¹COR¹,
alkyl(C₁-C₆)-S(O)ₙR², alkyl(C₁-C₆)-OR¹, alkyl(C₁-C₆)-CON(R¹)₂, alkyl (C₁-C₆)-SO₂N(R¹)₂, alkyl(C₁-C₆)-NR¹COR¹, alkyl(C₁-C₆)-NR¹SO₂R², alkyl (C₁-C₆) -phényle, alkyl(C₁-C₆)-hétéroaryle, alkyl(C₁-C₆)-hétérocyclyle, phényle, hétéroaryle ou hétérocyclyle, les 6 derniers radicaux étant substitués chacun par s radicaux choisis dans le groupe constitué par halogéno, nitro, cyano, alkyle en C₁-C₆, halogéno-alkyle(C₁-C₆) cycloalkyle en C₃-C₆, S(O)ₙ-alkyle(C₁-C₆), alcoxy en C₁-C₆, halogénoalcoxy(C₁-C₆), alcoxy (C₁-C₆)-alkyle(C₁-C₄) et cyanométhyle, et le groupe hétérocyclyle portant n groupes oxo,
Z représente un atome d'halogène, un groupe cyano, nitro, alkyle en C₁-C₆, alcoxy en C₁-C₆, halogéno-alkyle(C₁-C₆), cycloalkyle en C₃-C₆, S(O)ₙR², 1,2,4-triazol-1-yle, ou Z peut également représenter un atome d'hydrogène, lorsque Y représente le radical S(O)ₙR²,
V représente un atome d'hydrogène, un groupe alkyle en C₁-C₆, halogéno-alkyle (C₁-C₆), alcoxy en C₁-C₆, halogénoalcoxy(C₁-C₆), S(O)ₙ-alkyle(C₁-C₆), S(O)ₙ-halogéno-alkyle(C₁-C₆), alcoxy(C₁-C₆) -alkyle(C₁-C₄), un atome d'halogène, un groupe nitro ou cyano,
R, R' représentent chacun indépendamment l'un de l'autre un atome d'hydrogène, un groupe alkyle en C₁-C₆, cycloalkyle en C₃-C₇, halogéno-alkyle(C₁-C₆), alcoxy en C₁-C₆, halogénoalcoxy(C₁-C₆), cyano-alkyle(C₁-C₆), cyano, méthylsulfényle, méthylsulfinyle, méthylsulfonyle, acétylamino, un atome d'halogène, un groupe amino, méthoxyméthyle,
R¹ représente un atome d'hydrogène, un groupe alkyle en C₁-C₆, alcényle en C₂-C₆, alcynyle en C₂-C₆, cycloalkyle en C₃-C₆, cycloalkyl (C₃-C₆) -alkyle(C₁-C₆), alkyl(C₁-C₆)-O-alkyle(C₁-C₆), phényle, phényl-alkyle(C₁-C₆), hétéroaryle, alkyl(C₁-C₆)-hétéroaryle, hétérocyclyle, alkyl(C₁-C₆)-hétérocyclyle, alkyl(C₁-C₆)-O-hétéroaryle, alkyl(C₁-C₆)-O-hétérocyclyle, alkyl (C₁-C₆)-NR³-hétéroaryle ou alkyl (C₁-C₆)-NR³-hétérocyclyle, les 16 radicaux nommés en dernier étant substitués par s radicaux choisis dans le groupe constitué par cyano, halogéno, nitro, OR³, S(O)ₙR⁴, N(R³)₂, NR³OR³, COR³, OCOR³, NR³COR³, NR³SO₂R⁴, CO₂R³, CON(R³)₂ et alcoxy (C₁-C₄)-alcoxycarbonyle (C₂-C₆) et le groupe hétérocyclyle portant n groupes oxo,
R² représente un groupe alkyle en C₁-C₆, cycloalkyle en C₃-C₆ ou cycloalkyl (C₃-C₆)-alkyle (C₁-C₆), ces trois radicaux précités étant substitués par s radicaux choisis dans le groupe constitué par halogéno et OR³,
R³ représente un atome d'hydrogène ou un groupe alkyle en C₁-C₆,
R⁴ représente un groupe alkyle en C₁-C₆,
R⁶ et R⁷ représentent chacun indépendamment l'un de l'autre un groupe méthyle, éthyle ou n-propyle,
ou
R⁶ et R⁷ forment ensemble avec l'atome de soufre, auquel ils sont liés, un cycle à 5 ou 6 chaînons, saturé, qui outre les atomes de carbone et outre l'atome de soufre du groupe sulfoximino contient m atomes d'oxygène
R⁸ représente un groupe méthyle, éthyle ou n-propyle,
R⁹ représente un atome d'hydrogène ou un groupe cyano,
m représente 0 ou 1,
n représente 0, 1 ou 2,
s représente 0, 1, 2 ou 3,

4. Compositions herbicides, **caractérisées par** une teneur à activité herbicide en au moins un composé de formule (I) selon l'une quelconque des revendications 1 à 3.

5. Compositions herbicides selon la revendication 4, en mélange avec des adjuvants de formulation.

6. Compositions herbicides selon la revendication 4 ou 5, contenant au moins une autre substance à activité pesticide, choisie dans le groupe constitué par les insecticides, acaricides, herbicides, fongicides, phytoprotecteurs et régulateurs de croissance.

7. Compositions herbicides selon la revendication 6, contenant un phytoprotecteur.

8. Compositions herbicides selon la revendication 7, contenant du cyprosulfamide, du cloquintocet-mexyle, du méfenpyr-diéthyle ou de l'isoxadifène-éthyle.

9. Compositions herbicides selon l'une quelconque des revendications 6 à 8, contenant un autre herbicide.

10. Procédé pour la lutte contre des plantes indésirables, **caractérisé en ce qu'**on applique sur les plantes ou sur le lieu de la croissance de plantes indésirables une quantité efficace d'au moins un composé de formule (I) selon l'une quelconque des revendications 1 à 3 ou d'une composition herbicide selon l'une quelconque des revendications 4 à 9.

11. Utilisation de composés de formule (I) selon l'une quelconque des revendications 1 à 3 ou de compositions herbicides selon l'une quelconque des revendications 4 à 9, pour la lutte contre des plantes indésirables.

12. Utilisation selon la revendication 11, **caractérisée en ce que** les composés de formule (I) sont utilisés pour la lutte contre des plantes indésirables dans des cultures de plantes utiles.

13. Utilisation selon la revendication 12, **caractérisée en ce que** les plantes utiles sont des plantes utiles transgéniques.
